(19) Europäisches Patentamt
European Patent Office
Office européen des brevets

(11) **EP 1 591 112 A1**

(12) **EUROPEAN PATENT APPLICATION**
published in accordance with Art. 158(3) EPC

(43) Date of publication:
**02.11.2005 Bulletin 2005/44**

(21) Application number: **04707627.8**

(22) Date of filing: **03.02.2004**

(51) Int Cl.[7]: **A61K 31/121**, A61K 31/352, A61P 35/00, A61P 43/00, C07D 311/30, C07D 493/04, C07H 17/07, C12N 5/08, C12Q 1/02

(86) International application number:
**PCT/JP2004/001054**

(87) International publication number:
**WO 2004/069233 (19.08.2004 Gazette 2004/34)**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **04.02.2003 JP 2003026857**

(71) Applicant: **KABUSHIKI KAISHA YAKULT HONSHA**
**Minato-ku, Tokyo 105-8660 (JP)**

(72) Inventors:
- **YAMAZAKI, Ryuta,**
  **Kabushiki Kaisha Yakult Honsha**
  **Tokyo 1058660 (JP)**
- **NISHIYAMA, Yukiko,**
  **Kabushiki Kaisha Yakult Honsha**
  **Tokyo 1058660 (JP)**
- **FURUTA, Tomio,**
  **Kabushiki Kaisha Yakult Honsha**
  **Tokyo 1058660 (JP)**
- **MATSUZAKI, Takeshi,**
  **Kabushiki Kaisha Yakult Honsha**
  **Tokyo 1058660 (JP)**
- **HATANO, Hiroshi,**
  **Kabushiki Kaisha Yakult Honsha**
  **Tokyo 1058660 (JP)**
- **MATSUMOTO, Sachiko,**
  **Kabushiki Kaisha Yakult Honsha**
  **Tokyo 1058660 (JP)**
- **AIYAMA, Ritsuo,**
  **Kabushiki Kaisha Yakult Honsha**
  **Tokyo 1058660 (JP)**
- **YOSHIDA, Oh, Kabushiki Kaisha Yakult Honsha**
  **Tokyo 1058660 (JP)**
- **NAGAOKA, Masato,**
  **Kabushiki Kaisha Yakult Honsha**
  **Tokyo 1058660 (JP)**
- **HASHIMOTO, Shusuke,**
  **Kabushiki Kaisha Yakult Honsha**
  **Tokyo 1058660 (JP)**
- **SUGIMOTO, Yoshikazu**
  **Kashiwa-shi, Chiba 2770061 (JP)**

(74) Representative: **Hartz, Nikolai**
**Wächtershauser & Hartz**
**Weinstrasse 8**
**80333 München (DE)**

(54) **BREAST CANCER-RESISTANT PROTEIN INHIBITOR**

(57) The invention provides a cancer cell useful in screening BCRP-inhibitors, and a *BCRP*-inhibitor.

The BCRP-inhibitor contains, as the active ingredient, a flavonoid represented by any of the following formulae (1), (2), (3), (4), and (5):

EP 1 591 112 A1

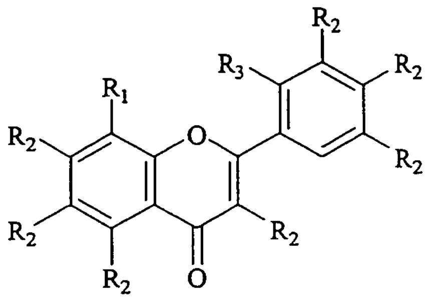

(1)

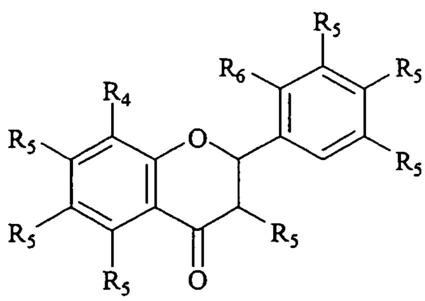

(2)

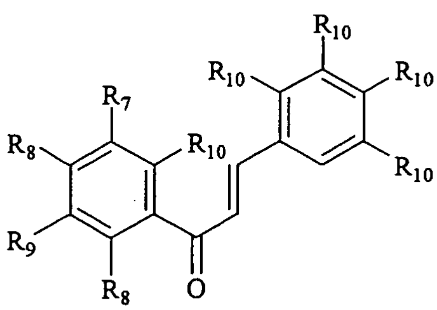

(3)



(Cont. next page)

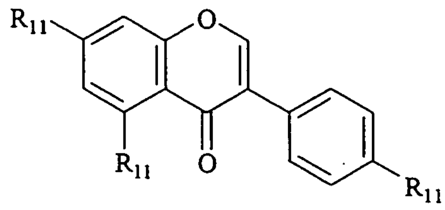

(4)

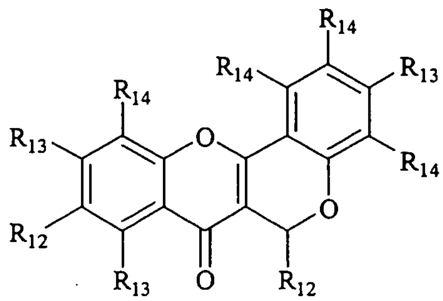

(5)

and glycosides, esters, or salts thereof; and an anticancer agent containing the *BCRP*-inhibitor together with an anticancer agent available as the substrate of *BCRP*.

## Description

Technical Field

The present invention relates to a breast cancer resistance protein (*BCRP*) inhibitor, and to an SN-38-resistant cancer cell line which is useful for screening *BCRP* inhibitors.

Background Art

Serious problems associated with cancer chemotherapy include intrinsic resistance to an anticancer drug, which invalidates the effect of the anticancer drug from the beginning of cancer therapy, and development of acquired resistance to an anticancer drug (i.e., reduction of the effect of the drug, which is caused by long-term continuous administration thereof). Overcoming such anticancer drug resistance has been envisaged to lead to improvement of the performance of cancer chemotherapy, and thus attempts have been made to elucidate various resistance mechanisms. Particularly, expression of a drug transporter, which actively transports an anticancer drug out of cancer cells, thereby reducing the amount of intracellular accumulation of the drug, is considered to play an important role in such a resistance mechanism.

Particularly, P-glycoprotein, which is a drug transporter discovered in the 1970s, and is encoded by an *MDR1* gene, has been considered a potent target molecule of a multidrug-resistance-overcoming agent, since this protein causes cross-resistance to a plurality of anticancer drugs having different chemical structures and action mechanisms. However, it has been gradually elucidated that a drug transporter other than P-glycoprotein is also associated with an anticancer drug resistance mechanism, and demand has arisen for development of a resistance-overcoming agent which targets such a drug transporter.

Under such circumstances, there was discovered, in 1998, breast cancer resistance protein (*BCRP*), which is a drug transporter belonging to a group which is called "ATP-binding cassette (ABC) transporter superfamily" to which P-glycoprotein also belongs (Proc. Natl. Acad. Sci. USA 95, 15665-15670 (1998)). *BCRP* has a structure including only one ATP-binding cassette, which differs from that of P-glycoprotein or another drug transporter, which has two ATP-binding cassettes. *BCRP* is intimately involved in the mechanism of resistance to a topoisomerase I inhibitor (e.g., irinotecan hydrochloride (CPT-11) or topotecan) or to a topoisomerase II inhibitor (e.g., mitoxantrone). Meanwhile, *BCRP* has been elucidated to exhibit substrate specificity different from that of P-glycoprotein, since *BCRP* does not act on, for example, paclitaxel or vincristine, which is excreted by P-glycoprotein, and *BCRP* is involved in excretion of a camptothecin derivative (e.g., CPT-11 or SN-38 (active metabolite of CPT-11)), which is barely excreted extracellularly by P-glycoprotein (Cancer Res. 59, 5938-5946 (1999)). In addition, *BCRP* has been suggested to be involved in the limitation of the bioavailability of an orally administered anticancer drug (J. Clin. Oncol. 20, 2943-2950 (2002)). In view of the foregoing, demand has arisen for development of a *BCRP* inhibitor, which is envisaged to exhibit the effect of overcoming anticancer drug resistance that is not overcome by a conventional resistance-overcoming agent, and to improve the bioavailability of an anticancer drug.

Hitherto, a variety of P-glycoprotein inhibitors have been developed for the purpose of overcoming anticancer drug resistance. However, since few BCRP-specific inhibitors have been reported, and such inhibitors have been considered to exhibit unsatisfactory BCRP-inhibiting effect, demand has arisen for a drug which exhibits more potent BCRP-inhibiting effect (Mol. Cancer. Ther. 1, 427-434 (2002)). Although some flavonoids have been reported to exhibit the effect of inhibiting P-glycoprotein (J. Med. Chem. 41, 4161-4164 (1998); Biochem. Biophys. Res. Commun. 295, 832-840 (2002)), a flavonoid exhibiting BCRP-inhibiting effect has not yet been known.

Objects of the present invention are to provide a cancer cell line which is useful for screening *BCRP* inhibitors, and to provide a *BCRP* inhibitor.

Disclosure of the Invention

In order to solve the aforementioned problems, the present inventors have subcultured A549 cell line, which is a cancer cell line derived from human non-small cell lung cancer, in an SN-38-containing medium, to thereby establish a human cancer cell line which has acquired anticancer drug resistance through overexpression of *BCRP.* In addition, on the basis of the effect of overcoming anticancer drug resistance, the present inventors have screened a variety of plant-derived components by use of the thus-established cancer cell line, and as a result have found that a flavonoid represented by the following formula (1), (2), (3), (4), or (5) exhibits potent BCRP-inhibiting effect. The present invention has been accomplished on the basis of this finding.

Accordingly, the present invention provides a *BCRP* inhibitor, an agent for overcoming anticancer drug resistance (hereinafter may be referred to as an "anticancer-drug-resistance-overcoming agent") for a cancer which has acquired *BCRP*-mediated resistance, or an anticancer-drug-effect-enhancing agent for a cancer which expresses

*BCRP* and exhibits low sensitivity to an anticancer drug, the *BCRP* inhibitor or anticancer-drug-resistance-enhancing agent containing, as an active ingredient, a flavonoid represented by the following formula (1), (2), (3), (4), or (5):

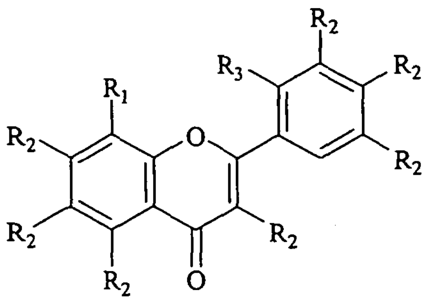

(1)

[wherein $R_1$ represents a hydrogen atom, a hydroxyl group, a halogen atom, a lower alkoxy group, or a lower alkyl group; each of seven $R_2$s, which may be identical to or different from one another, represents a hydrogen atom, a hydroxyl group, a lower alkoxy group, a lower alkyl group, a lower alkenyl group, or a sugar residue, or $R_1$ and the $R_2$ adjacent thereto may together form a pyran ring which may be substituted by a lower alkyl group; and $R_3$ represents a hydrogen atom, a hydroxyl group, a halogen atom, a lower alkyl group, a lower alkoxy group, an amino group, or a nitro group];

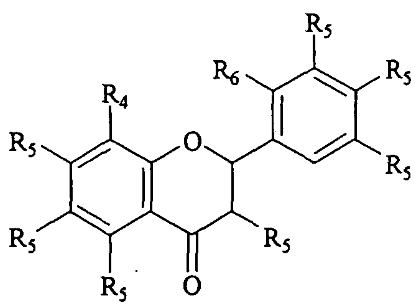

(2)

[wherein $R_4$ represents a hydrogen atom, a hydroxyl group, a halogen atom, a lower alkoxy group, a lower alkyl group, or a lower alkenyl group; each of seven $R_5$s, which may be identical to or different from one another, represents a hydrogen atom, a hydroxyl group, a lower alkoxy group, or a lower alkyl group, or two adjacent $R_5$s may together form a pyran ring which may be substituted by a lower alkyl group; and $R_6$ represents a hydrogen atom, a hydroxyl group, a halogen atom, a lower alkoxy group, a lower alkyl group, an amino group, or a nitro group];

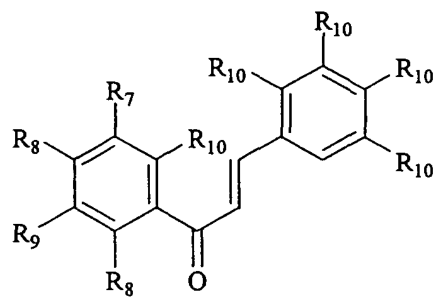

(3)

[wherein $R_7$ represents a hydrogen atom, a hydroxyl group, a halogen atom, a lower alkoxy group, or a lower alkyl group; each of two $R_8$s, which may be identical to or different from each other, represents a hydrogen atom, a hydroxyl group, a lower alkoxy group, or a lower alkyl group; $R_9$ represents a hydrogen atom, a hydroxyl group, a halogen atom, or a lower alkoxy group; and each of five $R_{10}$s, which may be identical to or different from one another, represents a hydrogen atom, a hydroxyl group, or a lower alkoxy group];

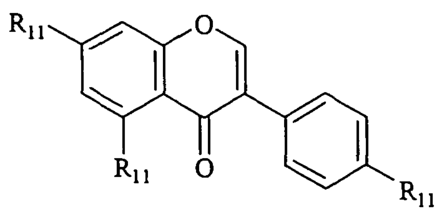

(4)

[wherein each of three $R_{11}$s, which may be identical to or different from one another, represents a hydrogen atom, a hydroxyl group, or a lower alkoxy group]; or

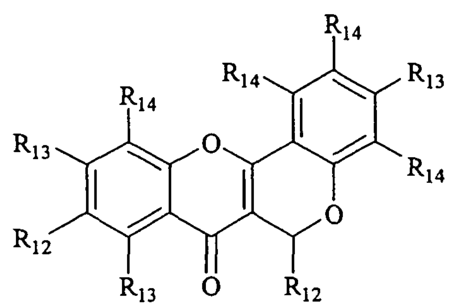

(5)

[wherein $R_{12}$ represents a hydrogen atom or a lower alkenyl group; $R_{13}$ represents a hydrogen atom or a hydroxyl group; and $R_{14}$ represents a hydrogen atom], a glycoside of the flavonoid, an ester of the flavonoid, or a salt of the flavonoid.

The present invention also provides an anticancer drug containing the aforementioned *BCRP* inhibitor, and an anticancer drug which can serve as a *BCRP* substrate.

The present invention also provides a novel flavonoid represented by the following formula (6):

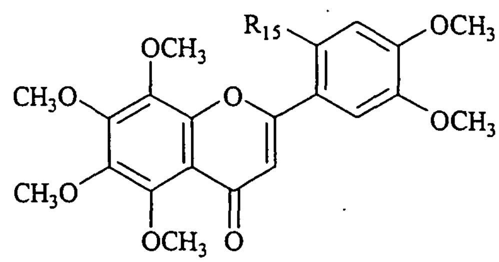

(6)

[wherein $R_{15}$ represents an amino group or a nitro group].

The present invention also provides an SN-38-resistant human non-small cell lung cancer A549 cell line which overexpresses *BCRP*.

Brief Description of the Drawings

Fig. 1 shows the level of resistance of A549/SN-38-4 cell line to SN-38 (A) or to mitoxantrone (B).
Fig. 2 shows the results of RT-PCR analysis of expression of mRNAs of various drug transporters in A549 cell line and A549/SN-38 cell lines.
Fig. 3 shows the results of real-time RT-PCR quantitative analysis of expression of mRNAs of *BCRP* (A) and MRP2 (B) in A549 cell line and A549/SN-38 cell lines.
Fig. 4 shows the amount of SN-38 (A) or SN-38-glucuronide (B) accumulated in A549 cell line and A549/SN-38-4 cell line.
Fig. 5 shows the effect of flavonoids [compound 1-1 (A), compound 1-4 (B), compound 1-6 (C), compound 1-14 (D), compound 3-4 (E), and compound 3-6 (F)] in overcoming SN-38 resistance of P388/BCRP cell line.
Fig. 6 shows the effect of flavonoids in increasing accumulation of SN-38 in P388/BCRP cell line.
Fig. 7 shows the effect of flavonoids in increasing accumulation of SN-38 in MCF-7 cell line.

Best Mode for Carrying Out the Invention

Human non-small cell lung cancer A549 cell line is readily cultured, can be implanted in mice, and is known to exhibit high sensitivity to SN-38, which is an active form of CPT-11 (J. Clin. Invest. 101, 1789-1796 (1998)). The A549 cell line was subcultured in a medium while the concentration of SN-38 contained in the medium was increased in a stepwise manner, to thereby establish an SN-38-resistant A549 cell line. As described below in Examples, the resultant SN-38-resistant A549 cell line, which has acquired SN-38 resistance by overexpressing *BCRP* and reducing intracellular accumulation of SN-38, is useful for screening *BCRP* inhibitors. The SN-38-resistant A549 cell line may be employed for in vitro screening, or may be employed for in vivo screening through implantation of the cell line in mice.

By use of the SN-38-resistant A549 cell line, a variety of plant-derived components have been screened on the basis of SN-38-resistance-overcoming effect, and as a result, a flavonoid has been found to exhibit potent BCRP-inhibiting effect.

The flavonoid employed in the present invention is a flavone derivative represented by formula (1), a flavanone derivative represented by formula (2), a chalcone derivative represented by formula (3), an isoflavone derivative represented by formula (4), or a flavonoid derivative represented by formula (5).

In formulas (1) through (4), the lower alkoxy group represented by $R_1$ to $R_{11}$ is preferably a C1-C4 alkoxy group, with a methoxy group being particularly preferred. The lower alkyl group represented by $R_1$ to $R_8$ is preferably a C1-C4 alkyl group, with a methyl group being particularly preferred. The lower alkenyl group represented by $R_2$, $R_4$, or $R_{12}$ of formula (1), (2), or (5) is preferably a C2-C5 alkenyl group, with a 3-methyl-1-butenyl group or a 3-methyl-2-butenyl group being particularly preferred. Examples of the halogen atom include a fluorine atom, a chlorine atom, a bromine atom, and an iodine atom, with a chlorine atom or a bromine atom being preferred. In formula (1), a pyran ring formed by $R_1$ and the $R_2$ adjacent thereto may be substituted by a C1-C4 lower alkyl group (in particular, a methyl group). In formula (2), two adjacent $R_5$s which form a pyran ring are preferably on a dihydrobenzopyran ring. A pyran

ring formed by two adjacent $R_5$s may be substituted by a C1-C4 lower alkyl group (in particular, a methyl group).

Among the compounds of formula (6), a compound in which $R_{15}$ is a nitro group (i.e., 2'-nitro-4',5,5',6,7,8-hexamethoxyflavone) is also called 2'-nitronobiletin, and a compound in which $R_{15}$ is an amino group (i.e., 2'-amino-4',5,5',6,7,8-hexamethoxyflavone) is also called 2'-aminonobiletin, and these two compounds are novel compounds.

The aforementioned flavonoid encompasses a glycoside obtained through addition of a sugar (e.g., β-D-glucoside). The flavonoid may be a pharmacologically acceptable salt formed through addition of, for example, sodium, potassium, or a hydrochloride, and the present invention also encompasses such a salt. The flavonoid may be a pharmacologically acceptable ester formed through addition of a lower fatty acid such as acetic acid, propionic acid, lactic acid, or butyric acid, and the present invention also encompasses such an ester. The flavonoid may be in the form of a solvate (e.g., a hydrate), and the present invention also encompasses such a solvate. The present invention also encompasses isomers of the flavonoid, and mixtures of such isomers.

No particular limitations are imposed on the origin of the flavonoid of the present invention, and the flavonoid may be derived from a plant, or may be a chemical synthetic product or a semi-synthetic product. No particular limitations are imposed on the method for obtaining the flavonoid of the present invention from a plant. Examples of the method include a method in which roots, stems, leaves, fruit, and/or flowers of a plant are subjected to extraction with water, a lower alcohol (e.g., methanol or ethanol), or a water-soluble organic solvent (e.g., acetone) at room temperature or an elevated temperature; a method in which such a plant portion is subjected to extraction with a mixture of water and such a water-soluble organic solvent; and a method in which such a plant portion is subjected to extraction with a mixture of a hydrophobic organic solvent (e.g., chloroform, dichloromethane, an acetic acid ester, toluene, or a supercritical fluid of carbon dioxide gas) and a water-soluble organic solvent (e.g., methanol). Particularly preferably, roots, stems, and/or leaves of a plant are chopped or ground, and the resultant product is subjected to extraction with a lower alcohol such as methanol. The thus-obtained extract can be further fractionated and purified by means of column chromatography or a similar technique, to thereby isolate the flavonoid of the present invention.

2'-Nitro-4',5,5',6,7,8-hexamethoxyflavone, which is a compound represented by formula (6) wherein $R_{15}$ is a nitro group, can be produced through nitration of 3',4',5,6,7,8-hexamethoxyflavone by means of a conventional method. A mixed acid (e.g., nitric acid-sulfuric acid) is employed as a nitrating agent.

2'-Amino-4',5,5',6,7,8-hexamethoxyflavone, which is a compound represented by formula (6) wherein $R_{15}$ is an amino group, can be produced through reduction of 2'-nitro-4',5,5',6,7,8-hexamethoxyflavone by means of a customary method.

The flavonoid of the present invention may be administered without any treatment. Alternatively, so long as the effects of the present invention are not reduced, the flavonoid may be mixed with a carrier which is generally employed for drug preparation, such as a dispersing aid or an excipient, and may be used in the form of an injection or a peroral preparation such as a powder, a solution, a capsule, a suspension, an emulsion, a syrup, an elixir, a granule, a pill, a tablet, a troche, or a lemonade.

Examples of such a carrier include water-soluble monosaccharides, oligosaccharides, and polysaccharides, such as mannitol, lactose, and dextran; gel-forming or water-soluble celluloses, such as hydroxypropyl cellulose, hydroxypropylmethyl cellulose, and methyl cellulose; water-absorbing and poorly water-soluble celluloses, such as crystalline cellulose, α-cellulose, cross-linked carboxymethylcellulose sodium, and derivatives thereof; water-absorbing and poorly water-soluble polysaccharides, such as hydroxypropyl starch, carboxymethyl starch, cross-linked starch, amylose, amylopectin, pectin, and derivatives thereof; water-absorbing and poorly water-soluble gums, such as gum arabi, tragacanth gum, glucomannan, and derivatives thereof; cross-linked vinyl polymers, such as polyvinyl pyrrolidone, cross-linked polyacrylic acid and salts thereof, cross-linked polyvinyl alcohol, polyhydroxyethyl methacrylate, and derivatives thereof; and molecular aggregate (e.g., liposome)-forming lipids, such as phosphorlipid and cholesterol.

When the flavonoid of the present invention exhibits low solubility, the compound may be subjected to solubilization. Examples of the solubilization technique include techniques which are generally applicable to drugs, such as a technique in which a surfactant (e.g., a polyoxyethylene alcohol ether, a polyoxyethylene acyl ester, a sorbitan acyl ester, or a polyoxyethylene sorbitan acyl ester) is added to the flavonoid, and a technique employing a water-soluble polymer (e.g., polyethylene glycol). If desired, there may be employed, for example, a technique for forming a soluble salt of the flavonoid, or a technique for forming a clathrate compound by use of cyclodextrin or a similar material. A solubilization technique may be appropriately selected in accordance with the target flavonoid.

The *BCRP* inhibitor may be employed as an anticancer-drug-resistance-overcoming agent for a cancer which has acquired *BCRP*-mediated resistance through administration of an anticancer drug. Meanwhile, the *BCRP* inhibitor may be employed as an anticancer-drug-effect-enhancing agent for a cancer which originally expresses *BCRP* and exhibits low sensitivity to an anticancer drug. No particular limitations are imposed on the target anticancer drug of an anticancer-drug-resistance-overcoming agent or anticancer-drug-effect-enhancing agent containing the *BCRP* inhibitor as an active ingredient, so long as the anticancer drug can serve as a *BCRP* substrate. Examples of such an anticancer drug include topoisomerase I inhibitors such as irinotecan hydrochloride/CPT-11 (active metabolite: SN-38) and topotecan; topoisomerase II inhibitors such as mitoxantrone, doxorubicin, daunorubicin, bisantrene, and etoposide;

and antifolates such as methotrexate.

The dose of the *BCRP* inhibitor of the present invention may be appropriately determined in accordance with, for example, the administration method or the symptom of a patient. The daily dose for an adult is preferably 1 mg to 10 g, more preferably 100 mg to 10 g, particularly preferably 500 mg to 10 g. No particular limitations are imposed on the ratio between an anticancer drug and the *BCRP* inhibitor, and the preferred ratio varies in accordance with, for example, the type of an anticancer drug or inhibitor to be employed. When, for example, irinotecan hydrochloride is employed as an anticancer drug, the ratio by weight of the anticancer drug to the *BCRP* inhibitor is preferably 1 : 1 to 1 : 500, particularly preferably 1 : 1 to 1 : 100, more preferably 1 : 1 to 1 : 10.

Specific examples of the flavonoid employed in the present invention are shown in Tables 1, 2, 3, 4, and 5.

Table 1

| Flavone derivatives | |
|---|---|
| Compound | Chemical name |
| 1-1 | 5,4'-Dihydroxy-7-methoxyflavone (product of EXTRASYNTHÉSE) |
| 1-2 | 5,7-Dihydroxy-3',4',5'-trimethoxyflavone (product of INDOFINE Chemical Company) |
| 1-3 | 5,7-Dihydroxyflavone (product of INDOFINE Chemical Company) |
| 1-4 | 3',4'-Dimethoxyflavone (product of EXTRASYNTHÉSE) |
| 1-5 | 2'-Amino-4',5,5',6,7,8-hexamethoxyflavone (Example 4) |
| 1-6 | 3',5-Dihydroxy-4',6,7-trimethoxyflavone (Example 1) |
| 1-7 | 3,4',5,7-Tetrahydroxyflavone (product of EXTRASYNTHÉSE) |
| 1-8 | 3',4',5,7-Tetrahydroxyflavone (product of EXTRASYNTHÉSE) |
| 1-9 | 4',5,7-Trihydroxyflavone (product of EXTRASYNTHÉSE) |
| 1-10 | 4',5-Dihydroxyflavone-7-glucoside (product of EXTRASYNTHÉSE) |
| 1-11 | 3,4',5,7-Tetrahydroxy-3'-methoxyflavone (product of EXTRASYNTHÉSE) |
| 1-12 | 5,6-Dihydroxy-7-methoxyflavone (product of EXTRASYNTHÉSE) |
| 1-13 | 3'-Hydroxy-4',5,6,7-tetramethoxyflavone (product of EXTRASYNTHÉSE) |
| 1-14 | 3',4',7-Trimethoxyflavone (product of INDOFINE Chemical Company) |
| 1-15 | 6,7-Dimethoxyflavone (product of INDOFINE Chemical Company) |
| 1-16 | 2',4',6-Trimethoxyflavone (product of INDOFINE Chemical Company) |
| 1-17 | 3,4'-Dimethoxyflavone (product of INDOFINE Chemical Company) |
| 1-18 | 6,8-Dichloro-4'-methylflavone (product of INDOFINE Chemical Company) |
| 1-19 | 5,4'-Dihydroxy-6,7-dimethoxyflavone (Example 2) |
| 1-20 | 2'-Nitro-4',5,5',6,7,8-hexamethoxyflavone (Example 3) |
| 1-21 | 3',6-Dimethoxyflavone (product of INDOFINE Chemical Company) |
| 1-22 | 4' ,5-Dihydroxy-3,3' ,7-trimethoxyflavone (Example 5) |
| 1-23 | 5-Hydroxy-3',4',7-trimethoxyflavone (Example 6) |
| 1-24 | 3,3',4',5.,6,7,8-Heptamethoxyflavone (Example 7) |
| 1-25 | 3',4',5,6,7-Pentamethoxyflavone (Example 7) |
| 1-26 | 5-Hydroxy-6,7-dimethoxyflavone-4'-glucoside (Example 8) |
| 1-27 | 5-Hydroxy-7-methoxyflavone-4'-glucoside (Example 9) |
| 1-28 | 4H,8H-Benzo[1,2-b:3,4-b']dipyran-4-one,2-(2,4-dihydroxyphenyl)-5-hydroxy-8,8-dimethyl-3-(3-methyl-2-butenyl) (Example 10) |
| 1-29 | 2',4',5-Trihydroxy-7-methoxy-6-(3-methyl-1-butenyl)-3-(3-methyl-2-butenyl)-flavone (Example 11) |

Table 2

| Flavanone derivatives | |
|---|---|
| Compound | Chemical name |
| 2-1 | 3',5,7-Trihydroxy-4'-methoxyflavanone (product of EXTRASYNTHÉSE) |
| 2-2 | 4'·5-Dihydroxy-7-methoxyflavanone (product of EXTRASYNTHÉSE) |
| 2-3 | 2H, 6H-Benzo [1, 2-b: 5, 4-b'] dipyran-6-one, 7, 8-dihydro-5-hydroxy-8-(4-hydroxyphenyl)-2,2-dimethyl-10-(3-methyl-2-butenyl) (Example 12) |

Table 3

| Chalcone derivatives | |
|---|---|
| Compound | Chemical name |
| 3-1 | 2',4-Dihydroxy-4',6'-dimethoxychalcone (product of EXTRASYNTHÉSE) |
| 3-2 | 3,4-Dimethoxychalcone (product of EXTRASYNTHÉSE) |
| 3-3 | 2'-Hydroxy-3,4-dimethoxychalcone (product of INDOFINE Chemical Company) |
| 3-4 | 2'-Hydroxy-3,4,4',6'-tetraethoxychalcone (product of INDOFINE Chemical Company) |
| 3-5 | 2',4'-Dihydroxy-3,4-dimethoxychalcone (product of INDOFINE Chemical Company) |
| 3-6 | 2'-Hydroxy-2,4,4',5,6'-pentamethoxychalcone (product of INDOFINE Chemical Company) |
| 3-7 | 5'-Bromo-3'-chloro-2'-hydroxy-3,4,5-trimethoxychalcone (product of INDOFINE Chemical Company) |
| 3-8 | 2',6'-Dihydroxy-4,4'-dimethoxychalcone (product of EXTRASYNTHÉSE) |
| 3-9 | 2'-Hydroxy-2,3,5'-trimethoxychalcone (product of INDOFINE Chemical Company) |
| 3-10 | 3'-Bromo-5'-Chloro-2'-hydroxy-3,4,5-trimethoxychalcone (product of INDOFINE Chemical Company) |

Table 4

| Isoflavone derivatives | |
|---|---|
| Compound | Chemical name |
| 4-1 | 5,7-Dihydroxy-4'-methoxyisoflavone (product of EXTRASYNTHÉSE) |
| 4-2 | 5-Hydroxy-4'-methoxyisoflavone-7-glucoside (product of EXTRASYNTHÉSE) |

Table 5

| Flavonoid derivative | |
|---|---|
| Compound | Chemical name |
| 5-1 | 6H, 7H- [1] benzopyrano [4, 3-b] [1] benzopyran-7-one,3,8,10-trihydroxy-9-(3-methyl-1-butenyl)-6-(2-methyl-1-propenyl) (Example 13) |

Examples

The present invention will next be described in more detail by way of Examples, which are provided for illustrative purposes only and therefore should not be construed as limiting the invention thereto.

Example 1: Isolation of 3',5-dihydroxy-4',6,7-trimethoxyflavone (compound 1-6)

Carqueja (crude drug produced in Brazil) (368 g) was refluxed with methanol for two hours, and the resultant

extract was brought to dryness under reduced pressure. An aliquot (5.09 g) of the resultant extract (8.1 g) was subjected to elution with hexane by means of medium-pressure chromatography, to thereby yield fraction A, followed by sequential elution with hexane-ethyl acetate (4 : 1) (fraction B), ethyl acetate (fraction C), and methanol (fraction D). An aliquot (1.0 g) of the above-obtained fraction C (1.53 g) was subjected to elution with hexane by means of medium-pressure chromatography under monitoring at 254 nm, followed by sequential elution with hexane-ethyl acetate (2 : 1), hexane-ethyl acetate (1 : 1), ethyl acetate, and methanol, to thereby yield 12 fractions. The fraction C6 (100.1 mg) obtained through elution with hexane-ethyl acetate (2 : 1) was analyzed by means of high-performance liquid chromatography [Mightysil RP-18 GP, 6.0 × 250 mm, eluted with acetonitrile-water (45 : 55)]. As a result, the fraction C6 exhibited a peak at a retention time of 15.2 min. The fraction C6 was recrystallized from chloroform-methanol (1 : 1), to thereby yield 47.9 mg of compound 1-6. The results of analysis of this compound are as follows.
IR νmax (KBr) $cm^{-1}$: 1649(C=O)
EI-MS m/z: 315$[M]^+$
$^1$H-NMR (DMSO-$d_6$) δ: 3.74(C6-$OCH_3$, s), 3.88(C4'-$OCH_3$, s), 3.94(C7-$OCH_3$, s), 6.83(C3-H, s), 6.92(C8-H, s), 7.10 (C5'-H, d, J=8.8Hz), 7.48(C2'-H, d, J=2.2Hz), 7.57(8.5, C6'-H, dd, J=2.4Hz), 9.44(C3'-OH, s), 12.90 (C5-OH, s),
$^{13}$C-NMR (DMSO-$d_6$) δ: 56.2 (C6-$OCH_3$), 56.9 (C7-$OCH_3$), 60.5 (C6-$OCH_3$), 92.0 (C-8), 103.7(C-3), 105.4 (C-10), 112.5 (C-5'), 113.2 (C-2'), 119.3 (C-6'), 123.2 (C-1'), 132.2(C-6), 147.0 (C-3'), 151.7 (C-4'), 152.0(C-5), 153.1(C-9), 159.1(C-7), 164.3(C-2), 182.5(C-4)

Example 2: Isolation of 5,4'-dihydroxy-6,7-dimethoxyflavone (compound 1-19)

*Artemisia capillaries flos* (The capitula of *Artemisia capillaries*) *flos* obtained from Takasago Yakugyo Co., Ltd. (500 g) was refluxed with methanol for two hours, and the resultant extract was brought to dryness under reduced pressure. An aliquot (5.07 g) of the resultant extract (40.1 g) was applied to silica gel, and then subjected to elution with hexane by means of medium-pressure chromatography, to thereby yield fraction A, followed by sequential elution with hexane-ethyl acetate (4 : 1) (fraction B), ethyl acetate (fraction C), and methanol (fraction D). An aliquot (1.5 g) of the above-obtained fraction C (1.56 g) was subjected to elution with chloroform by means of medium-pressure chromatography under monitoring at 254 nm, followed by sequential elution with chloroform-methanol (99 : 1), chloroform-methanol (19 : 1), chloroform-methanol (9 : 1), and methanol, to thereby yield 14 fractions. The fraction C6 (112.4 mg) obtained through elution with chloroform was dissolved in methanol, and a methanol-insoluble fraction C6 (70 mg) was obtained. The methanol-insoluble fraction C6 was analyzed by means of high-performance liquid chromatography [Mightysil RP-18 GP, 6.0 × 250 mm, eluted with an acetonitrile-0.7% formic acid solution (30 : 70) mixture]. As a result, the fraction exhibited a peak at a retention time of 28.0 min. The fraction exhibiting a peak at a retention time of 28.0 min was purified, to thereby yield compound 1-19. The results of analysis of this compound are as follows.
IR νmax (KBr) $cm^{-1}$: 1655(C=O)
MS(ESI) m/z: 315$[M+H]^+$,
$^1$H-NMR ($CDCl_3$) δ: 3.87($OCH_3$, s), 3.99($OCH_3$, s), 6.57(C3-H, s), 6.62(C8-H, s), 6.96(C3'-H, C5'-H, dd, J=8.8Hz, 1.7), 7.79(C2'-H, C6'-H, dd, J=8.8Hz, 1.5), 9.89(C4'-OH, s), 12.88 (C5-OH, s)

Example 3: Synthesis of 2'-nitro-4',5,5',6,7,8-hexamethoxyflavone (compound 1-20)

3',4',5,6,7,8-Hexamethoxyflavone (831 mg) was dissolved in concentrated sulfuric acid (17 mL) in an ice bath. A 5% fuming nitric acid-sulfuric acid mixture (1.7 mL) was added dropwise to the resultant solution, followed by stirring in the ice bath for 30 minutes. The resultant reaction mixture was added to ice water (50 mL), followed by stirring for 10 minutes. The thus-precipitated crystals were subjected to suction filtration, and the resultant crystals were washed with ethyl acetate. The aqueous layer was subjected to extraction with ethyl acetate (50 mL × 2). The thus-obtained ethyl acetate layer was concentrated to dryness under reduced pressure. The resultant solid product and the above-obtained crystals were combined and subjected to fractionation by means of silica gel column chromatography [$SiO_2$ produced by Kanto Kagaku, eluted with hexane-ethyl acetate (1 : 1)]. The target fraction was concentrated to dryness under reduced pressure, to thereby yield 2'-nitro-4',5,5',6,7,8-hexamethoxyflavone (326 mg, yield: 35%).
IR νmax (KBr) $cm^{-1}$ 3489, 2943, 2838, 1655, 1530
MS(ESI) m/z : 448 $[M+H]^+$
$^1$H-NMR($CDCl_3$) δ: 3.83(3H, s), 3.95 (3H, s), 3.97(3H, s), 3.98(3H, s), 4.03(3H, s), 4.08(3H, s), 6.41(1H, s), 7.00(1H, s), 7.67(1H, s)

Example 4: Synthesis of 2'-amino-4',5,5',6,7,8-hexamethoxyflavone (compound 1-5)

2'-Nitro-4',5,5',6,7,8-hexamethoxyflavone (135 mg) was dissolved in a concentrated hydrochloric acid-methanol (1 : 1) mixture (5 mL), and iron powder (51.6 mg) was added to the resultant solution, followed by stirring at room

temperature for four hours. The resultant reaction mixture was partitioned between purified water (20 mL) and ethyl acetate (20 mL). The pH of the thus-obtained aqueous layer was adjusted to 8 by use of a saturated aqueous solution of sodium hydrogencarbonate, and the resultant aqueous layer was subjected to extraction with ethyl acetate (130 mL × 2). The resultant ethyl acetate layer was washed with saturated brine (70 mL), and then dried over anhydrous magnesium sulfate. The magnesium sulfate was separated through filtration, and the filtrate was washed with ethyl acetate. Thereafter, the resultant ethyl acetate layer was concentrated to dryness under reduced pressure. The thus-obtained solid product was subjected to fractionation by means of silica gel column chromatography [$SiO_2$ produced by Kanto Kagaku, eluted with hexane-ethyl acetate (1 : 1)]. The target fraction was concentrated to dryness under reduced pressure, to thereby yield 2'-amino-4',5,5',6,7,8-hexamethoxyflavone (66 mg, yield: 52%).
IR vmax(KBr) $cm^{-1}$ : 3496, 3326, 1624, 1560, 1520
MS(ESI) m/z : 418 $[M+H]^+$
$^1H$-NMR($CDCl_3$) δ: 3.85(3H, s), 3.89(3H, s), 3.96(6H, s), 3.99(3H, s), 4.09(3H, s), 6.28(1H, s), 6.48(1H, s), 6.96(1H, s)

Example 5: Isolation of 4',5-dihydroxy-3,3',7-trimethoxyflavone (compound 1-22)

Branches and leaves of *Pogostemon cablin* (crude drug produced in Thailand) (53.8 g) were subjected to extraction with methanol at room temperature for one week, and the resultant extract was brought to dryness under reduced pressure. An aliquot (4.7 g) of the resultant extract (4.9 g) was subjected to elution with hexane by means of medium-pressure chromatography, to thereby yield fraction A, followed by sequential elution with hexane-ethyl acetate (4 : 1) (fraction B), ethyl acetate (fraction C), and methanol (fraction D). An aliquot (0.5 g) of the above-obtained fraction B (1.3 g) was subjected to elution with chloroform by means of centrifugal chromatography under monitoring at 254 nm, followed by sequential elution with chloroform-methanol (9 : 1) and methanol, to thereby yield 18 fractions. The fraction B9 (14.8 mg) obtained through elution with the chloroform-methanol (9 : 1) was analyzed by means of high-performance liquid chromatography (Mightysil RP-18 GP, 6.0 × 250 mm, eluted with acetonitrile-water (45 : 55)). As a result, this fraction exhibited a peak at a retention time of 14.5 min. The results of analysis of the thus-obtained compound are as follows.
MS(ESI) m/z: 345 $[M+H]^+$, 343 $[M-H]^-$
$^1H$-NMR ($CDCl_3$) δ: 3.85($C_{3'}$-$OCH_3$, s), 3.88($C_{7'}$-$OCH_3$, s), 3.98($C_3$-$OCH_3$, s), 6.36($C_6$-H, d, *J*=2.4), 6.44($C_8$-H, d, *J*=2.4), 7.09($C_{5'}$-H, d, *J*=8.8), 7.67($C_{6'}$-H, dd, *J*=8.8, 2.0), 7.70($C_{2'}$-H, d, J=2.0
$^{13}C$-NMR ($CDCl_3$) δ: 55.8 ($C_{3'}$-$OCH_3$), 56.1($C_7$-$OCH_3$), 60.2($C_3$-$OCH_3$), 92.2(C-8), 97.8(C-6), 106.0(C-10), 110.9(C-2'), 114.6(C-5'), 122.4(C-1'), 122.7(C-6'), 138.8(C-3), 146.3(C-3'), 148.3(C-4'), 155.9(C-2), 156.7(C-5), 162.0(C-9), 165.4(C-7), 178.7(C-4)

Example 6: Isolation of 5-hydroxy-3',4',7-trimethoxyflavone (compound 1-23)

The whole plant of *Striga asiatica* (crude drug produced in China) (about 300 g) was subjected to reflux extraction with methanol for two hours, and the resultant extract was brought to dryness under reduced pressure. An aliquot (5.0 g) of the resultant extract (6.8 g) was subjected to elution with hexane by means of medium-pressure chromatography, to thereby yield fraction A, followed by sequential elution with hexane-ethyl acetate (4 : 1) (fraction B), ethyl acetate (fraction C), and methanol (fraction D). The above-obtained fraction B (480 mg) was subjected to elution with chloroform by means of centrifugal chromatography under monitoring at 254 nm, followed by sequential elution with chloroform-methanol (9 : 1) and methanol, to thereby yield nine fractions. The fractions B3 and B4 (42.3 mg each) obtained through elution with chloroform were analyzed by means of high-performance liquid chromatography (Mightysil RP-18 GP, 6.0 × 250 mm, eluted with acetonitrile-methanol-water (45 : 5 : 50)), and a compound exhibiting a peak at a retention time of 16.5 min was isolated. The results of analysis of this compound are as follows.
MS(ESI)m/z: 329$[M+H]^+$
$^1H$-NMR ($CDCl_3$) δ: 3.89, 3.97, 3.99 ($C_7$, $C_{3'}$ , $C_{4'}$ -$OCH_3$, s), 6.38($C_6$-H, d, *J*=2.4), 6.50($C_8$-H, d, *J*=2.4), 6.59($C_3$-H, s), 6.98($C_{5'}$-H, d, *J*=8.8), 7.34($C_{2'}$-H, d, *J*=2.4), 7.53($C_{6'}$-H, dd, *J*=8.8, 2.4), 12.79($C_5$-OH, s)
$^{13}C$-NMR ($CDCl_3$) δ: 55.8 ($C_{-7}$-$OCH_3$), 56.1 ($C_{3'}$-$OCH_3$, $C_{4'}$-$OCH_3$), 92.7 (C-2), 98.1(C-8), 104.7(C-3), 105.6(C-10), 108.9(C-2'), 111.2(C-5'), 120.1(C-6'), 123.8(C-1'), 149.3(C-3'), 152.3(C-4'), 151.7(C-4'), 157.7(C-9), 162.2(C-2), 164.0 (C-5), 165.5(C-7), 182.4(C-4)

Example 7: Isolation of 3,3',4',5,6,7,8-heptamethoxyflavone (compound 1-24) and 3',4',5,6,7-pentamethoxyflavone (compound 1-25)

The pericarp of *Citrus tangerina* (crude drug produced in China) (about 2 Kg) was subjected to reflux extraction with methanol for two hours, and the resultant extract was brought to dryness under reduced pressure. An aliquot (35.2 g) of the resultant extract (356.5 g) was subjected to elution with hexane by means of medium-pressure chromatography, to thereby yield fraction A, followed by sequential elution with hexane-ethyl acetate (4 : 1) (fraction B), ethyl acetate (fraction C), and methanol (fraction D). The above-obtained fraction C (556 mg) was subjected to elution with chloroform by means of medium-pressure silica gel column chromatography under monitoring at 254 nm, followed by sequential elution with chloroform-methanol (98 : 2), chloroform-methanol (95 : 5), and methanol, to thereby yield 10 fractions. The fractions C3 and C4 (total: 230 mg) obtained through elution with chloroform were subjected to elution with hexane by means of medium-pressure silica gel column chromatography under monitoring at 254 nm, followed by sequential elution with hexane-ethyl acetate (19 : 1), hexane-ethyl acetate (9 : 1), hexane-ethyl acetate (8 : 2), hexane-ethyl acetate (7 : 3), hexane-ethyl acetate (5 : 5), hexane-ethyl acetate (1 : 2), ethyl acetate, and methanol, to thereby yield 10 fractions. The fraction C23G (90.0 mg) obtained through elution with hexane-ethyl acetate (5 : 5), and the fraction C23J (5.2 mg) obtained through elution with hexane-ethyl acetate (1 : 2) were analyzed by means of high-performance liquid chromatography (Mightysil RP-18 GP, 6.0 $\times$ 250 mm, eluted with acetonitrile-5% formic acid-methanol-water (30 : 10 : 20 : 40)). As a result, there were obtained a fraction exhibiting a peak at a retention time of 18.3 min (compound 1-24) and a fraction exhibiting a peak at a retention time of 10.6 min (compound 1-25). The results of analysis of these compounds are as follows. Compound 1-24
MS(ESI) m/z: 433$[M+H]^+$
$^1$H-NMR ($CDCl_3$) $\delta$: 3.91($OCH_3$, s), 3.96($OCH_3$, s), 3.98($OCH_3$$\times$2, s), 3.99($OCH_3$, s), 4.02($OCH_3$, s), 4.11($OCH_3$, s), 7.03($C_5$,-H, d, *J*=8.3), 7.82($C_2$,-H, d, *J*=2.4), 7.85($C_6$,-H, dd, *J*=8.3, 2.4) $^{13}$C-NMR ($CDCl_3$) $\delta$: 56.6, 56.7($C_{3'}$ , $C_4$,-$OCH_3$), 60.5, 62.4, 62.5, 62.7, 63.0($C_3$, $C_5$, $C_6$, $C_7$, $C_8$-$OCH_3$) , 107.4(C-2'), 111.7(C-5'), 115.2(C-10), 122.7(C-6'), 124.1(C-1'), 138.6(C-8), 141.5(C-6), 144.6(C-3), 147.4(C-5), 148.9(C-9), 149.5(C-3'), 151.8(C-2), 152.0(C-7), 153.8(C-4'), 174.6 (C-4)
Compound 1-25
MS(ESI) m/z: 373 $[M+H]^+$, 371 $[M-H]^-$
$^1$H-NMR ($CDCl_3$) $\delta$: 3.93($OCH_3$, s), 3.96($OCH_3$, s), 3.98($OCH_3$, s), 3.99($OCH_3$$\times$2, s), 6.60($C_3$-H, s), 6.80 ($C_8$-H, s), 6.97 ($C_5$,-H, d, *J*=8.8), 7.33($C_2$,-H, d, *J*=2.0), 7.51 ($C_6$,-H, *J*=8.3, 2.0) $^{13}$C-NMR ($CDCl_3$) $\delta$: 56.3, 56.3, 56.5($C_7$, $C_{3'}$ , $C_4$,-$OCH_3$), 61.7, 62.5($C_5$, $C_6$-$OCH_3$), 96.4 (C-8), 107.6(C-3), 108.8(C-2'), 111.3 (C-5'), 112.9(C-10), 119.8(C-6'), 124.3 (C-1'), 140.4(C-6), 139.3(C-3'), 152.0(C-4'), 152.6(C-9), 154.7(C-5), 157.8(C-7), 161.3(C-2), 177.4(C-4)

Example 8: Isolation of 5-hydroxy-6,7-dimethoxyflavone-4'-glucoside (compound 1-26)

The stems and leaves of *Cirsium kamtschaticum* (produced in Hokkaido, Japan) (55.0 g) were subjected to extraction with methanol at room temperature for one week, and the resultant extract was brought to dryness under reduced pressure. An aliquot (5.1 g) of the resultant extract (13.5 g) was subjected to elution with hexane by means of medium-pressure chromatography, to thereby yield fraction A, followed by sequential elution with hexane-ethyl acetate (4 : 1) (fraction B), ethyl acetate (fraction C), and methanol (fraction D). An aliquot (2.0 g) of the above-obtained fraction D (3.8 g) was subjected to elution with chloroform by means of medium-pressure silica gel column chromatography under monitoring at 254 nm, followed by sequential elution with chloroform-methanol (9 : 1), chloroform-methanol (8 : 1), chloroform-methanol (3 : 7), and methanol, to thereby yield 10 fractions. The fraction D4 (1.34 g) obtained through elution with chloroform-methanol (9 : 1) was recrystallized from chloroform, to thereby yield white crystals. The white crystals were analyzed by means of high-performance liquid chromatography (Mightysil RP-18 GP, 6.0 $\times$ 250 mm, eluted with acetonitrile-water (20 : 80)). As a result, a compound exhibiting a peak at a retention time of 27.6 min was obtained. The results of analysis of this compound are as follows.
MS(ESI) m/z: 477$[M+H]^+$
$^1$H-NMR (DMSO-d6) $\delta$: 3.74($C_7$-$CH_3$, s), 3.94 ($C_6$,-$CH_3$, s), 4.59 (t, J=5.6), 5.04(d, *J*=7.8), 5.06(d, *J*=5.6), 5.13(d, *J*=4.4), 5.37(d, *J*=4.6), 6.98($C_8$-H, s), 6.96($C_3$-H, s), 7.20 ($C_3$,-H, $C_5$,-H, d, J=9.0), 8.07 ($C_2$,-H, $C_6$,-H, *d*, J=9.0), 12.9($C_5$-OH, s) $^{13}$C-NMR (DMSO-d6) $\delta$: 56.5($C_7$-$OCH_3$), 60.0($C_6$-OCH3), 60.6(C-6''), 69.6(C-4''), 73.1(C-2''), 76.5(C-3''), 77.2(C-5''), 91.7(C-8), 99.8(C-1''), 103.6(C-3), 105.2(C-10), 116.6(C-3', C-5'), 123.8(C-1'), 128.2(C-2', C-6'), 131.9(C-6), 152.0 (C-5), 152.7(C-9), 158.7(C-7), 160.3(C-4'), 163.4(C-2), 182.3(C-4)

Example 9: Synthesis of 5-hydroxy-7-methoxyflavone-4'-glucoside (compound 1-27)

Compound 1-1 (5,4'-dihydroxy-7-methoxyflavone) (100 mg) was dissolved in acetone, and $\alpha$-D-acetobromoglucose (647 mg) and potassium carbonate (231 mg) were added to the resultant solution, followed by reflux under boiling for a whole day and night under vigorous stirring. The resultant mixture was subjected to filtration, to thereby remove insoluble matter. The thus-collected product was washed with chloroform and mixed with the above-obtained filtrate, and the resultant mixture was concentrated to dryness under reduced pressure. The resultant residue was purified by means of silica gel chromatography employing a chloroform/methanol system. The thus-purified product was suspended in anhydrous methanol, and 28% NaOMe was added dropwise to the resultant suspension, followed by stirring at room temperature for 0.5 h. Water was added to the resultant reaction mixture, and the mixture was

neutralized with ion-exchange resin (sulfonic acid-$^+$H type). The resin was removed through filtration, and the mixture was concentrated to dryness under reduced pressure, followed by purification by means of silica gel chromatography employing a chloroform/methanol system, to thereby yield the target product (10 mg, yield: 6%).
Pale yellow crystal, MS(ESI) m/z:447[M+H]$^+$
$^1$H-NMR(DMSO-d6)δ3.18(1H, t, J=9Hz), 3.28(1H t, J=9Hz), 3.32(1H t, J=9Hz), 3.41(1H, m), 3.50(1H, dd, J=6Hz, 12Hz), 3.71(1H, dd, J=2Hz, 11Hz), 3.88(3H, s), 5.04(1H, d, J=7Hz), 6.40 (1H, d, J=2Hz), 6.83 (1H, d, J=2Hz), 6.94(1H, s), 7.21(2H, d, J=9Hz) , 8.07(2H, d, J=9Hz)

Example 10: Isolation of 4H,8H-benzo[1,2-b:3,4-b']dipyran-4-one,2-(2,4-dihydroxyphenyl)-5-hydroxy-8,8-dimethyl-3-(3-methyl-2-butenyl) (compound 1-28)

Jack Fruit Tree (the xylem of *Artocarpus heterophyllus*, crude drug produced in Thailand) (169 g) was subjected to extraction with methanol at room temperature for one week, and the resultant extract was brought to dryness under reduced pressure. An aliquot (4.7 g) of the resultant extract (8.1 g) was subjected to elution with hexane by means of medium-pressure chromatography, to thereby yield fraction A, followed by sequential elution with hexane-ethyl acetate (4 : 1) (fraction B), ethyl acetate (fraction C), and methanol (fraction D). The above-obtained fraction C (3.2 g) was subjected to elution with chloroform by means of medium-pressure silica gel column chromatography under monitoring at 254 nm, followed by sequential elution with chloroform-methanol (98 : 2), chloroform-methanol (19 : 1), chloroform-methanol (9 : 1), chloroform-methanol (4 : 1), chloroform-methanol (2 : 1), chloroform-methanol (1 : 1), and methanol, to thereby yield 10 fractions. The fraction C4 (217 mg) obtained through elution with chloroform was analyzed by means of high-performance liquid chromatography (Mightysil RP-18 GP, 6.0 × 250 mm, eluted with acetonitrile-water (70 : 30)), and a compound exhibiting a peak at a retention time of 9.8 min (compound 1-28) was isolated. The results of analysis of this compound are as follows.
Compound 1-28
MS (ESI) m/z: 421[M+H]$^+$, 419[M-H]$^-$
$^1$H-NMR ($CD_3OD$) δ: 1.36($CH_3$, s), 1.43($CH_3$×2, s), 1.58 ($CH_3$, s), 3.08 (H, d, J=6.8), 5.09(H, tt, *J*=6.8, 3.9), 5.66($C_9$-H, d, *J*=9.8), 6.25 ($C_6$-H, s), 6.39 ($C_5$,-H, dd, *J*=8.3, 2.4), 6.40 ($C_3$,-H, s), 6.67 ($C_{10}$-H, d, *J*=9.8), 7.06($C_{6'}$-H, d, *J*=8.3)
$^{13}$C-NMR ($CD_3OD$) δ: 24.6($CH_3$), 25.6($CH_3$), 28.3($C_8$-$CH_3$×2), 78.7 (C-8), 95.4 (C-6), 103.5(C-3'), 105.8, 107.7(C-9), 113.0(C-1'), 116.1(C-5'), 121.8(C-3), 122.5, 132.2(C-10), 132.5, 157.0, 157.5(C-2'), 158.7(C-4'), 160.3(C-7), 161.7(C-2), 163.4 (C-5), 183.6(C-4)

Example 11: Isolation of 2',4',5-trihydroxy-7-methoxy-6-(3-methyl-1-butenyl)-3-(3-methyl-2-butenyl)-flavone (compound 1-29)

Jack Fruit Tree (the xylem of *Artocarpus heterophyllus*, crude drug produced in Thailand) (169 g) was subjected to extraction with methanol at room temperature for one week, and the resultant extract was brought to dryness under reduced pressure. An aliquot (4.7 g) of the resultant extract (8.1 g) was subjected to elution with hexane by means of medium-pressure chromatography, to thereby yield fraction A, followed by sequential elution with hexane-ethyl acetate (4 : 1) (fraction B), ethyl acetate (fraction C), and methanol (fraction D).

The fraction C4 (217 mg) obtained through elution with chloroform was analyzed by means of high-performance liquid chromatography (Mightysil RP-18 GP, 6.0 × 250 mm, eluted with acetonitrile-water (70 : 30)), and a compound exhibiting a peak at a retention time of 8.5 min (compound 1-29) was isolated. The results of analysis of this compound are as follows.
Compound 1-29
MS(ESI) m/z: 437[M+H]$^+$, 435 [M+H]$^-$
$^1$H-NMR ($CD_3OD$) δ: 1.02($C_{17}$, $C_{18}$-$CH_3$, d, *J*=6.83), 1.33($C_{12}$-$CH_3$, s), 1.52 ($C_{13}$-$CH_3$, s), 2.33($C_{16}$-H, m), 3.02, 3.04 ($C_9$-$CH_2$, s) , 3.77($C_7$-$OCH_3$, s), 5.04($C_{10}$-H, tt, *J*=5.4, 1.5), 6.33($C_8$-H, s), 6.34($C_5$,-H, d, *J*=9.3), 6.36($C_3$,-H, d, *J*=3.4), 6.43($C_{14}$-H, dd, *J*=16.1, 1.0), 6.56($C_{15}$-H, dd, *J*=16.1, 7.3), 7.02($C_6$,-H, *J*=8.3)
$^{13}$C-NMR($CD_3OD$)δ: 18.5(C-13), 24.1(C-17, C-18), 25.8(C-9), 26.7(C-12), 35.2(C-16), 57.3($C_7$-$OCH_3$), 91.5(C-8), 104.6(C-3'), 106.7(C-4a), 108.8(C-5'), 111.2(C-6), 114.1(C-1'), 118.0(C-14), 122.9(C-3), 123.7(C-10), 133.4(C-11), 133.5(C-6'), 143.5(C-15), 158.6(C-2'), 158.6(C-8a), 160.4(C-5), 162.6(C-4'), 164.3(C-2), 165.0(C-7), 184.6(C-4)

Example 12: Isolation of 2H,6H-benzo[1,2-b:5,4-b']dipyran-6-one,7,8-dihydro-5-hydroxy-8-(4-hydroxyphenyl)-2,2-dimethyl-10-(3-methyl-2-butenyl) (compound 2-3)

Branches of *Albizzia myriophylla* (crude drug produced in Thailand) (60.0 g) were subjected to extraction with methanol at room temperature for one week, and the resultant extract was brought to dryness under reduced pressure. An aliquot (3.80 g) of the resultant extract (4.4 g) was subjected to elution with hexane by means of medium-pressure

chromatography, to thereby yield fraction A, followed by sequential elution with hexane-ethyl acetate (4 : 1) (fraction B), ethyl acetate (fraction C), and methanol (fraction D). The above-obtained fraction B (1.2 g) was analyzed by means of high-performance liquid chromatography (Mightysil RP-18 GP, 6.0 × 250 mm, eluted with acetonitrile-water (70 : 30)). As a result, a compound exhibiting a peak at a retention time of 15.2 min was obtained. The results of analysis of this compound are as follows.
MS(ESI) m/z: 407$[M+H]^+$, 405 $[M-H]^-$
$^1$H-NMR($CDCl_3$)δ: 1.43, 1.45($C_{2,,,}$-$CH_3$×2, s), 1.65($C_{3,,}$-$CH_3$×2, s), 2.80($C_3$-H, dd, *J*=17.1, 3.2), 3.39($C_3$-H, dd, *J*=17.1, 2.9), 3.21($C_{1''}$-$H_2$, d, *J*=7.3), 5.14($C_{2''}$-H, t, *J*=7.3), 5.34($C_2$-H, dd, *J*=12.7, 2.9), 5.50 ($C_3$-''' H, d, *J*=10.0), 6.63 ($C_{4'''}$-H, d, *J*=10.0), 6.87($C_{3'}$-H, $C_{5'}$-H, d, J=8.8), 7.32 ($C_{2'}$-H, $C_{6'}$ -H, d, J=8.8)
$^{13}$C-NMR ($CDCl_3$) δ: 17.8(C-$_{5''}$-$CH_3$), 21.5 (C-1''), 25.8 ($C_{4''}$-$CH_3$) , 28.3(C-$_{6'''}$-CH3), 28.4(C-$_{5'''}$-CH3), 43.2(C-3), 78.1 (C-2'''), 78.5(C-2), 102.7(C-10), 102.8(C-6), 108.6(C-8), 115.5(C-3', C-5'), 115.7(C-4'''), 122.5(C-2''), 126.0(C-1'), 127.7 (C-2', C-6'), 131.1(C-3'''), 155.8(C-4'), 156.6(C-9), 159.3 (C-5), 160.0(C-7), 196.4(C-4)

Example 13: Isolation of 6H, 7H-[1]benzopyrano[4,3-b][1]benzopyran-7-one,3,8,10-trihydroxy-9-(3-methyl-1-butenyl)-6-(2-methyl-1-propenyl) (compound 5-1)

Jack Fruit Tree (the xylem of *Artocarpus heterophyllus*, crude drug produced in Thailand) (169 g) was subjected to extraction with methanol at room temperature for one week, and the resultant extract was brought to dryness under reduced pressure. An aliquot (4.7 g) of the resultant extract (8.1 g) was subjected to elution with hexane by means of medium-pressure chromatography, to thereby yield fraction A, followed by sequential elution with hexane-ethyl acetate (4 : 1) (fraction B), ethyl acetate (fraction C), and methanol (fraction D). The above-obtained fraction C (3.2 g) was subjected to elution with chloroform by means of medium-pressure silica gel column chromatography under monitoring at 254 nm, followed by sequential elution with chloroform-methanol (98 : 2), chloroform-methanol (19 : 1), chloroform-methanol (9 : 1), chloroform-methanol (4 : 1), chloroform-methanol (2 : 1), chloroform-methanol (1 : 1), and methanol, to thereby 10 fractions. The fraction C3 obtained through elution with chloroform was analyzed by means of high-performance liquid chromatography (Mightysil RP-18 GP, 6.0 × 250 mm, eluted with acetonitrile-water (70 : 30)), and a compound exhibiting a peak at a retention time of 10.8 min (compound 5-1) was isolated. The results of analysis of this compound are as follows.
Compound 5-1
MS(ESI) m/z: 421$[M+H]^+$
$^1$H-NMR($CD_3OD$)δ:1.92(CH3×2, d, *J*=6.6), 1.70(CH3, d, *J*=1.2), 1.95(CH3, d, *J*=1.2), 2.41(CH, m), 5.40(CH=, dt, *J*=9.5, 1.2), 6.14($C_6$-H, d, *J*=9.3), 6.30($C_2$-H, d, *J*=2.2), 6.42($C_{11}$-H, s), 6.50($C_4$-H, dd, J=8.5, 2.2), 6.53(=CH, dd, *J*=16.1, 1.0), 6.68(CH=, dd, *J*=16.1, 7.0), 7.59 ($C_5$-H, *d*, J=8.5) $^{13}$C-NMR($CD_3OD$)δ:19.3 (CH3), 23.9 (CH3×2), 26.6(CH3), 35.0, 67.3, 71.3, 95.0(C-11), 105.6(C-4), 106.0, 109.3, 110.9, 111.6, 118.0(C-2), 123.2, 126.8(C-5), 140.4, 143.2, 156.9, 157.6(C-5), 160.1, 161.2(C-8), 163.6(C-3), 165.3(C-10), 180.4(C-7)

Example 14: Establishment of SN-38-resistant A549 cell line

Human non-small cell lung cancer A549 cell line was subcultured at 5% $CO_2$ and 37°C by use of a Ham's F-12 medium containing 10% FBS, 100 U/mL of penicillin, and 100 μg/mL of streptomycin (10% FBS/Ham's F-12). SN-38-resistant A549 cell lines were selected by subculturing the A549 cell line in a medium for two months while the concentration of SN-38 contained in the medium was increased in a stepwise manner (4 to 10 ng/mL). Subsequently, the resultant SN-38-resistant A549 cell lines were subjected to cloning by means of limiting dilution, to thereby establish six cloned SN-38-resistant A549 cell lines (A549/SN-38-1 to 6).

Example 15: Anticancer drug sensitivity test of A549/SN-38 cell line

The A549 cell line or each of the A549/SN-38-1 to 6 was suspended in 10% FBS/Ham's F-12, and the resultant suspension was inoculated into a 96-well microplate ($2 \times 10^3$ cells/50μL/well), followed by culturing at 5% $CO_2$ and 37°C overnight. Thereafter, a solution of an anticancer drug in 10% FBS/Ham's F-12 (50 μL) was added to each of the wells, followed by culturing at 5% $CO_2$ and 37°C for 48 hours. After completion of culturing, the number of viable cells was counted by use of a viable cell counting reagent [TetraColor ONE (trade name), product of Seikagaku Corporation] according to the attached instruction manual. Table 6 and Fig. 1 show the sensitivities of the A549 cell line and the six A549/SN-38 cell lines to various anticancer drugs. "$IC_{50}$" corresponds to the concentration of an anticancer drug required for 50% inhibition of cell growth. "Relative resistance value" is obtained by dividing the $IC_{50}$ for an A549/SN-38 cell line by the $IC_{50}$ for the A549 cell line. The greater the relative resistance value, the higher the level of acquired resistance. The A549/SN-38 cell lines exhibited particularly strong resistance to SN-38 and mitoxantrone, which are *BCRP* substrates.

Table 6

| Anticancer drug | A549 | A549/SN-38 | | | | | |
|---|---|---|---|---|---|---|---|
| | | 1 | 2 | 3 | 4 | 5 | 6 |
| | $IC_{50}$ μg/mL | | | | | | |
| | (Relative resistance value) | | | | | | |
| SN-38 | 0.013 | 0.12 (9.2) | 0.10 (7.5) | 0.13 (9.7) | 0.10 (7.7) | 0.10 (7.7) | 0.10 (7.4) |
| Paclitaxel | 0.0049 | 0.0098 (2.0) | 0.012 (2.4) | 0.013 (2.7) | 0.014 (2.8) | 0.0061 (1.2) | 0.0071 (1.4) |
| Etoposide | 0.90 | 0.27 (0.3) | 0.80 (1.0) | 1.6 (1.9) | 3.5 (4.0) | 2.3 (2.7) | 1.0 (1.1) |
| Cisplatin | 18.6 | 15.1 (0.8) | 16.5 (0.9) | 15.1 (0.8) | 17.0 (0.9) | 15.9 (0.9) | 16.8 (0.9) |
| 5-Fluorouracil | 2.1 | 4.3 (2.0) | 1.3 (0.6) | 0.69 (0.3) | 0.91 (0.4) | 1.7 (0.8) | 1.0 (0.5) |
| Gemcitabine | 0.0090 | 0.0039 (0.4) | 0.0053 (0.6) | 0.0040 (0.4) | 0.0032 (0.4) | 0.0040 (0.4) | 0.0040 (0.4) |
| Doxorubicin | 0.10 | 0.19 (1.9) | 0.14 (1.4) | 0.12 (1.3) | 0.22 (2.3) | 0.057 (0.6) | 0.049 (0.5) |
| Mitomycin C | 0.11 | 0.13 (1.2) | 0.11 (1.1) | 0.0029 (0.3) | 0.15 (1.4) | 0.11 (1.0) | 0.047 (0.4) |

Example 16: RT-PCR analysis of A549/SN-38 cell line

RT-PCR was performed for analyzing the expression of mRNA of a drug transporter in the A549 cell line, the six A549/SN-38 cell lines, and human breast cancer MCF-7 cell line, which is known to express *BCRP.* The total RNA was extracted from cells by use of an RNA extraction reagent [ISOGEN (trade name), product of Nippon Gene Co., Ltd.], and RT-PCR was performed by use of an RT-PCR reagent [Ready·To·Go RT-PCR Beads (trade name), product of Amersham pharmacia biotech] and a thermal cycler [iCycler (trade mane), product of BIO-RAD] according to the attached instruction manual (total RNA: 0.5 μg). The resultant PCR product was subjected to electrophoresis by use of 2% agarose gel, and then staining with ethidium bromide, followed by detection by use of a transilluminator. In addition, real-time RT-PCR was performed by use of a real-time RT-PCR reagent [SYBR Green RT-PCR Reagents (trade name), product of Applied Biosystems] and a sequence detection system [ABI PRISM 7000 (trade name), product of Applied Biosystems] according to the attached instruction manual, for quantitative analysis (total RNA: 0.1 μg). PCR primers corresponding to *BCRP*, *MDR1*, *MRP1*, *MRP2*, *MRP3, and G3PDH* (endogenous control gene) were designed on the basis of the following known mRNA base sequences (accession Nos. AF098951, AF016535, L05628, U63970, AF009670, and M33197), respectively. Fig. 2 shows the results of the RT-PCR, and Fig. 3 shows the results of the real-time RT-PCR. Expression of *BCRP* was remarkably increased in all the six A549/SN-38 cell lines, as compared with the case of the A549 cell line. In contrast, no significant difference was observed in the level of expression of MRP2 (whose substrate is SN-38, which is also a *BCRP* substrate) between the A549 cell line and the A549/SN-38 cell lines. Meanwhile, no significant difference was observed in the level of expression of the other drug transporters between the A549 cell line and the A549/SN-38 cell lines. These results suggest that *BCRP* is involved in the anticancer drug resistance mechanism of the A549/SN-38 cell lines. Through the above RT-PCR analysis, expression of *BCRP* was found in the human breast cancer MCF-7 cell line. In addition to the studies on expression of the aforementioned drug transporters, expression of topoisomerase-I, topoisomerase-II, Bcl-2, Bax, or IκBα was studied by means of western blotting, and topoisomerase-I activity was studied on the basis of DNA relaxation reaction. However, there were not obtained data suggesting that these proteins are involved in the anticancer drug resistance mechanism of the A549/SN-38 cell lines.

Example 17: Amount of anticancer drug accumulated in A549/SN-38 cell line

The A549 cell line or the A549/SN-38-4 cell line ($4 \times 10^6$ cells/mL) was suspended in 10% FBS/RPMI1640 (1 mL), and an SN-38 DMSO solution (1 μL, final concentration: 300 ng/mL) was added to the resultant suspension, followed by incubation at 37°C for 60 minutes. Thereafter, centrifugation was performed (2°C, 1,400 × g, 1 min), and the resultant supernatant was removed. Ice-cooled PBS was added to the thus-precipitated cells, and the cells were resuspended therein, followed by centrifugation (2°C, 1,400 × g, 1 min) for washing of the cells. This washing procedure was performed again, followed by addition of PBS (375 μL) and sonication of the cells. To the resultant destructed cells, methanol (375 μL) and 10% zinc sulfate solution (15 μL) were added, and the resultant mixture was stirred, followed by centrifugation (2°C, 12,500 × g, 5 min) and collection of the supernatant. The thus-collected supernatant was dispensed in a white 96-well microplate for fluorescence intensity measurement (200 μL/well), and then the amounts of SN-38 and an SN-38-glucuronide contained in the supernatant were measured by use of a microplate fluorometer [SPECTRA max GEMINI XS (trade name), product of Molecular Devices] (SN-38: excitation wavelength 380 nm, emission wavelength 560 nm; SN-38-glucuronide: excitation wavelength 370 nm, emission wavelength 430 nm), to thereby calculate the amount of intracellular accumulation of SN-38 and the glucuronide. As is clear from the results shown in Fig. 4, the amount of SN-38 accumulated in the A549/SN-38-4 cell line is about 1/5 that of SN-38 accumulated in the A549 cell line. The results support that *BCRP* is involved in the anticancer drug resistance mechanism of the A549/SN-38 cell lines. In contrast, virtually no SN-38-glucuronide was detected in both the A549 cell line and the A549/SN-38-4 cell line, indicating that the glucuronidation is not involved in the anticancer drug resistance mechanism.

Example 18: Effect of flavonoid in overcoming anticancer drug resistance of A549/SN-38 cell line

The A549 cell line or the A549/SN-38-4 cell line was suspended in 10% FBS/Ham's F-12, and the resultant suspension was inoculated into a 96-well microplate, followed by culturing at 5% $CO_2$ and 37°C overnight ($2 \times 10^3$ cells/50 μL/well). Thereafter, a solution of a flavonoid and SN-38 in 10% FBS/Ham's F-12 (25 μL) was added to each of the wells, followed by culturing at 5% $CO_2$ and 37°C for 48 hours. After completion of culturing, the number of viable cells was counted by use of TetraColor ONE according to the attached instruction manual. Table 7 shows the resistance overcoming effect of each of the tested flavonoids, which is represented by $EC_{50}$. "$EC_{50}$" corresponds to the concentration of a flavonoid required for 50% reduction of the relative resistance value. The results reveal that each of the flavonoids exhibits potent effect of overcoming SN-38 resistance of the A549/SN-38-4 cell line. When the concentration of the flavonoid falls within the range such that the compound overcomes the SN-38 resistance in a concentration-dependent manner, the flavonoid per se does not affect growth of the A549 cell line and the A549/SN-38-4 cell line. The results suggest that the flavonoid of the present invention inhibits *BCRP,* and overcomes the anticancer drug resistance of cancer cells.

Table 7

| Compound | Resistance overcoming effect $EC_{50}$ (μg/mL) |
|---|---|
| 1-1 | 0.017 |
| 1-2 | 0.048 |
| 1-3 | 0.10 |
| 1-4 | 0.030 |
| 1-5 | 0.081 |
| 1-6 | 0.017 |
| 1-7 | 0.13 |
| 1-8 | 0.46 |
| 1-9 | 0.12 |
| 1-10 | 0.17 |
| 1-11 | 0.16 |
| 1-12 | 0.074 |
| 1-13 | 0.11 |
| 1-14 | 0.0078 |
| 1-15 | 0.16 |
| 1-16 | 0.098 |

Table 7 (continued)

| Compound | Resistance overcoming effect $EC_{50}$ (μg/mL) |
|---|---|
| 1-17 | 0.060 |
| 1-18 | 0.14 |
| 1-19 | 0.015 |
| 1-20 | 0.75 |
| 1-21 | 0.52 |
| 1-22 | 0.046 |
| 1-23 | 0.0070 |
| 1-24 | 0.054 |
| 1-25 | 0.53 |
| 1-26 | 0.14 |
| 1-27 | 0.042 |
| 1-28 | 2.0 |
| 1-29 | 0.23 |
| 2-1 | 0.15 |
| 2-2 | 0.48 |
| 2-3 | 0.067 |
| 3-1 | 0.040 |
| 3-2 | 0.23 |
| 3-3 | 0.16 |
| 3-4 | 0.023 |
| 3-5 | 0.040 |
| 3-6 | 0.0074 |
| 3-7 | 0.65 |
| 3-8 | 0.79 |
| 3-9 | 0.94 |
| 3-10 | 0.97 |
| 4-1 | 0.33 |
| 4-2 | 0.84 |
| 5-1 | 0.18 |

Example 19: Effect of flavonoid in enhancing sensitivity of MCF-7 cell line to anticancer drug

The effect of a flavonoid on the sensitivity of cancer cells to an anticancer drug was studied by use of human breast cancer MCF-7 cell line, which is known to express *BCRP* (Blood 99, 3763-3770 (2002)). The MCF-7 cell line was suspended in 10% FBS/RPMI1640, and the resultant suspension was inoculated into a 96-well microplate, followed by culturing at 5% $CO_2$ and 37°C overnight ($3 \times 10^3$ cells/50 μL/well). Thereafter, a solution of a flavonoid and SN-38 in 10% FBS/RPMI1640 (25 μL) was added to each of the wells, followed by culturing at 5% $CO_2$ and 37°C for 48 hours. After completion of culturing, the number of viable cells was counted by use of TetraColor ONE according to the attached instruction manual. Table 8 shows change in the sensitivity of the MCF-7 cell line to SN-38, which change is caused by a flavonoid, by use of $IC_{50}$ (i.e., the concentration of SN-38 required for 50% inhibition of cell growth). The results reveal that each of the tested flavonoids enhances the sensitivity of the MCF-7 cell line to SN-38. When the concentration of the flavonoid falls within the range such that the compound enhances the SN-38 sensitivity in a concentration-dependent manner, the flavonoid *per se* does not affect growth of the MCF-7 cell line. The results suggest that the flavonoid of the present invention inhibits *BCRP*, and enhances the sensitivity of cancer cells to an anticancer drug.

Table 8

| Compound | Compound concentration (μg/mL) | | |
|---|---|---|---|
| | 0 | 0.1 | 1.0 |
| | SN-38: $IC_{50}$ (μg/mL) | | |
| 1-1 | 0.021 | 0.014 | 0.0063 |
| 1-2 | 0.014 | 0.013 | < 0.003 |
| 1-4 | 0.037 | 0.012 | 0.0061 |
| 1-14 | 0.024 | 0.015 | 0.0077 |
| 1-19 | 0.021 | 0.014 | 0.0038 |
| 2-1 | 0.031 | 0.022 | 0.011 |
| 3-1 | 0.016 | 0.015 | 0.0085 |
| 3-4 | 0.027 | 0.032 | 0.013 |
| 3-6 | 0.059 | 0.023 | 0.011 |

Example 20: Effect of flavonoid in overcoming anticancer drug resistance of human *BCRP*-transduced mouse leukemia P388 cell line

Mouse leukemia P388 cell line or human *BCRP*-transduced P388 cell line (P388/*BCRP* cell line, obtained from Yoshikazu Sugimoto, The Cancer Chemotherapy Center of Japanese Foundation for Cancer Research) was suspended in 10% FBS/RPMI1640, and the resultant suspension was inoculated into a 96-well microplate ($1 \times 10^4$ cells/50 μL/well). Thereafter, a solution of a flavonoid and SN-38 in 10% FBS/RPNI1640 (25 μL) was added to each of the wells, followed by culturing at 5% $CO_2$ and 37°C for 48 hours. After completion of culturing, the number of viable cells was counted by use of TetraColor ONE according to the attached instruction manual. The results are shown in Fig. 5. Each of the tested flavonoids exhibited potent effect of overcoming the SN-38 resistance of the P388/BCRP cell line. In contrast, the flavonoid did not affect the sensitivity of the P388 cell line to SN-38. The results demonstrate that the flavonoid of the present invention has *BCRP*-inhibiting effect.

Example 21: Effect of flavonoid on multidrug resistance of MES-SA/Dx5 cell line

Human uterine cancer MES-SA cell line or MES-SA/D×5 cell line which had acquired multidrug resistance through overexpression of P-glycoprotein (Cancer Res. 45, 4091-4096 (1985)) was suspended in 10% FBS/DMEM, and the resultant suspension was inoculated into a 96-well microplate, followed by culturing at 5% $CO_2$ and 37°C overnight ($3 \times 10^3$ cells/50 μL/well). Thereafter, a solution of a flavonoid and paclitaxel in 10% FBS/DMEM (25 μL) was added to each of the wells, followed by culturing at 5% $CO_2$ and 37°C for 48 hours. After completion of culturing, the number of viable cells was counted by use of TetraColor ONE according to the attached instruction manual. Table 9 shows the effect of each of the tested flavonoids on multidrug resistance by use of $EC_{50}$. "$EC_{50}$" corresponds to the concentration of a flavonoid required for 50% reduction of the relative resistance value. The results show that when the concentration of the flavonoid falls within the range employed for the test, the compound does not affect the paclitaxel resistance of the MES-SA/Dx5 cell line. In addition, the flavonoid per se did not affect growth of the MES-SA cell line and the MES-SA/Dx5 cell line. The results indicate that the flavonoid of the present invention does not act on P-glycoprotein, and has *BCRP* specificity.

Table 9

| Compound | Resistance overcoming effect $EC_{50}$ (μg/mL) |
|---|---|
| 1-1 | > 1.0 |
| 1-2 | > 1.0 |
| 1-3 | > 1.0 |
| 1-4 | > 1.0 |
| 1-5 | > 1.0 |
| 1-6 | > 1.0 |
| 1-7 | > 1.0 |
| 1-8 | > 1.0 |

Table 9 (continued)

| Compound | Resistance overcoming effect $EC_{50}$ (μg/mL) |
|---|---|
| 1-9 | > 1.0 |
| 1-10 | > 1.0 |
| 1-11 | > 1.0 |
| 1-12 | > 1.0 |
| 1-13 | > 1.0 |
| 1-14 | > 1.0 |
| 1-15 | > 1.0 |
| 1-16 | > 1.0 |
| 1-17 | > 1.0 |
| 1-18 | > 1.0 |
| 1-19 | > 1.0 |
| 1-20 | > 1.0 |
| 1-21 | > 1.0 |
| 2-1 | > 1.0 |
| 2-2 | > 1.0 |
| 3-1 | > 1.0 |
| 3-2 | > 1.0 |
| 3-3 | > 1.0 |
| 3-4 | > 1.0 |
| 3-5 | > 1.0 |
| 3-6 | > 1.0 |
| 3-7 | > 1.0 |
| 3-8 | > 1.0 |
| 3-9 | > 1.0 |
| 3-10 | > 1.0 |
| 4-1 | > 1.0 |
| 4-2 | > 1.0 |

Example 22: Effect of flavonoid on amount of anticancer drug accumulated in *BCRP*-expressing cell line

The P388 cell line or the P388/BCRP cell line ($1 \times 10^7$ cells/mL), or the MCF-7 cell line ($3 \times 10^6$ cells/mL) was suspended in 10% FBS/RPMI1640 (1 mL), and a flavonoid and SN-38 (final concentration: 500 ng/mL) were added to the resultant suspension, followed by incubation at 37°C for 60 minutes. Thereafter, centrifugation was performed (2°C, 1,400 × g, 1 min), and the resultant supernatant was removed. Ice-cooled 10% FBS/RPMI1640 was added to the thus-precipitated cells, and the cells were resuspended therein, followed by centrifugation (2°C, 1,400 × g, 1 min) for washing of the cells. This washing procedure was performed again, followed by addition of PBS (375 μL) and sonication of the cells. To the resultant destructed cells, methanol (375 μL) and 10% zinc sulfate solution (15 μL) were added, and the resultant mixture was stirred, followed by centrifugation (2°C, 12,500 × g, 5 min) and collection of the supernatant. The thus-collected supernatant was dispensed in a white 96-well microplate for fluorescence intensity measurement (200 μ/well), and then the amount of SN-38 contained in the supernatant was measured by use of a microplate fluorometer (SN-38: excitation wavelength 380 nm, emission wavelength 560 nm), to thereby calculate the amount of intracellular accumulation of SN-38. As is clear from Fig. 6, the flavonoid of the present invention increases the amount of accumulation of SN-38 in the P388/*BCRP* cell line. In addition, as is clear from Fig. 7, the flavonoid of the present invention increases the amount of accumulation of SN-38 in the MCF-7 cell line. The results suggest that the flavonoid of the present invention inhibits *BCRP,* and increases the amount of intracellular accumulation of an anticancer drug.

Example 23: Effect of flavonoid in overcoming anticancer drug resistance *in vivo*

Groups of six-week-old female $CDF_1$ mice, each group consisting of 5 mice, were employed. The P388 cell line or the P388/*BCRP* cell line ($1 \times 10^6$ cells) was implanted into the peritoneal cavity of each of the mice. From one day to 10 days after the tumor implantation, a flavonoid and irinotecan hydrochloride (CPT-11) were administered

intraperitoneally once a day (total number of administration: 10). Before administration, the flavonoid was suspended in a mixture of ethanol, polyoxyethylene (20) sorbitan monooleate [Tween 80 (trade name), product of Tokyo Kasei Kogyo Co., Ltd.], and 5% glucose (ethanol/Tween 80/5% glucose = 5 : 5 : 90), and CPT-11 was dissolved in saline. Merely the solvent was administered to mice in the control group. In order to evaluate the antitumor effect of the flavonoid, survival rate T/C (%) was calculated from the survival days of the tumor-implanted mice by use of the following formula.

$$\text{Survival rate T/C (\%)} = \text{(the mean survival days of mice in the treated flavonoid group)} \div \text{(the mean survival days of mice in the control group)} \times 100$$

The results are shown in Table 10. The results reveal that the flavonoid of the present invention inhibits *BCRP in vivo*, and exhibits the effect of overcoming anticancer drug resistance.

Table 10

| Compound | Dose (mg/kg/day) | | Survival days | T/C |
|---|---|---|---|---|
| | Compound | CPT-11 | Mean ± S.D. | (%) |
| 1-14 | 100 | 20 | 18.4 ± 4.5 | 153 |
| 1-15 | 100 | 20 | 18.2 ± 3.9 | 152 |
| 3-5 | 100 | 20 | 18.4 ± 0.5 | 153 |
| Solvent | 0 | 20 | 14.6 ± 0.5 | 122 |
| Solvent | 0 | 0 | 12.0 ± 0.7 | 100 |

Example 24: The following ingredients were mixed together, and the resultant mixture was formed into tablets.

Table 11

| | |
|---|---|
| Compound 1-1 | 100 mg |
| Lactose | 100 mg |
| Potato starch | 39 mg |
| Microcrystalline cellulose | 30 mg |
| Synthetic aluminum silicate | 30 mg |
| Calcium stearate | 1 mg |
| Total (for one tablet) | 300 mg |

The *BCRP* inhibitor of the present invention can overcome *BCRP*-associated anticancer drug resistance. In addition, the *BCRP* inhibitor can enhance the effect of an anticancer drug on *BCRP*-expressing cancer. Furthermore, the BCRP inhibitor is envisaged to improve the bioavailability of an anticancer drug, and to improve the performance of cancer chemotherapy.

## Claims

**1.** A breast cancer resistance protein inhibitor containing, as an active ingredient, a flavonoid represented by the following formula (1), (2), (3), (4), or (5) :

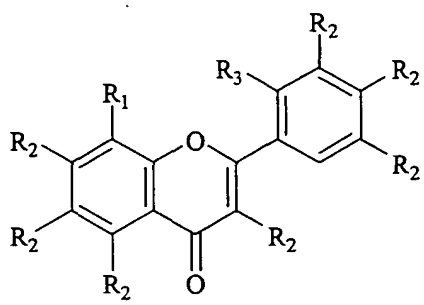

(1)

(wherein $R_1$ represents a hydrogen atom, a hydroxyl group, a halogen atom, a lower alkoxy group, or a lower alkyl group; each of seven $R_2$s, which may be identical to or different from one another, represents a hydrogen atom, a hydroxyl group, a lower alkoxy group, a lower alkyl group, a lower alkenyl group, or a sugar residue, or $R_1$ and the $R_2$ adjacent thereto may together form a pyran ring which may be substituted by a lower alkyl group; and $R_3$ represents a hydrogen atom, a hydroxyl group, a halogen atom, a lower alkyl group, a lower alkoxy group, an amino group, or a nitro group);

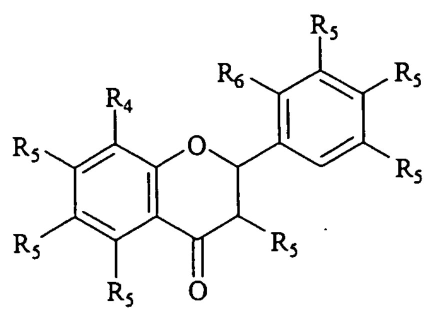

(2)

(wherein $R_4$ represents a hydrogen atom, a hydroxyl group, a halogen atom, a lower alkoxy group, a lower alkyl group, or a lower alkenyl group; each of seven $R_5$s, which may be identical to or different from one another, represents a hydrogen atom, a hydroxyl group, a lower alkoxy group, or a lower alkyl group, or two adjacent $R_5$s may together form a pyran ring which may be substituted by a lower alkyl group; and $R_6$ represents a hydrogen atom, a hydroxyl group, a halogen atom, a lower alkoxy group, a lower alkyl group, an amino group, or a nitro group);

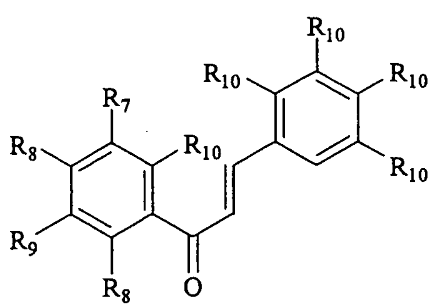

(3)

(wherein $R_7$ represents a hydrogen atom, a hydroxyl group, a halogen atom, a lower alkoxy group, or a lower alkyl

group; each of two $R_8$s, which may be identical to or different from each other, represents a hydrogen atom, a hydroxyl group, a lower alkoxy group, or a lower alkyl group; Rq represents a hydrogen atom, a hydroxyl group, a halogen atom, or a lower alkoxy group; and each of five $R_{10}$s, which may be identical to or different from one another, represents a hydrogen atom, a hydroxyl group, or a lower alkoxy group);

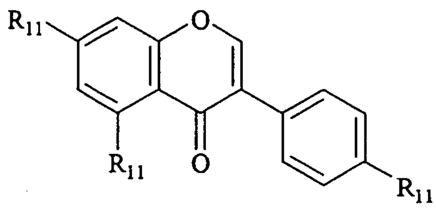

(4)

(wherein each of three $R_{11}$s, which may be identical to or different from one another, represents a hydrogen atom, a hydroxyl group, or a lower alkoxy group); or

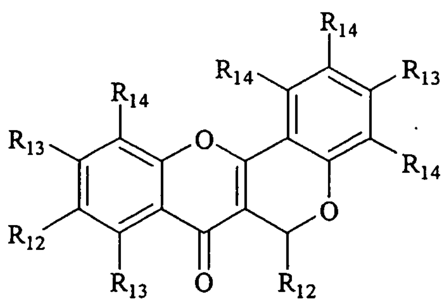

(5)

(wherein $R_{12}$ represents a hydrogen atom or a lower alkenyl group; $R_{13}$ represents a hydrogen atom or a hydroxyl group; and $R_{14}$ represents a hydrogen atom), a glycoside of the flavonoid, an ester of the flavonoid, or a salt of the flavonoid.

**2.** An anticancer-drug-resistance-overcoming agent for a cancer which has acquired *BCRP*-associated resistance, or an anticancer-drug-effect-enhancing agent for a cancer which expresses *BCRP* and exhibits low sensitivity to an anticancer drug, which agent contains, as an active ingredient, the flavonoid (1), (2), (3), (4), or (5) as described in claim 1, a glycoside thereof, an ester thereof, or a salt thereof.

**3.** An anticancer drug containing the flavonoid (1), (2), (3), (4), or (5) as described in claim 1, a glycoside thereof, an ester thereof, or a salt thereof; and an anticancer drug which can serve as a *BCRP* substrate.

**4.** A flavonoid represented by the following formula (6) :

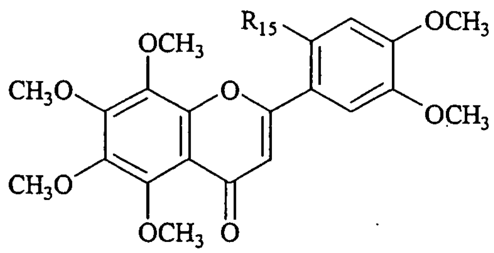

(6)

(wherein $R_{15}$ represents an amino group or a nitro group).

**5.** The flavonoid according to claim 4, wherein $R_{15}$ of formula (6) is a nitro group.

**6.** The flavonoid according to claim 4, wherein $R_{15}$ of formula (6) is an amino group.

**7.** A 7-ethyl-10-hydroxycamptothecin (SN-38)-resistant human non-small cell lung cancer A549 cell line which over-expresses *BCRP*.

Fig. 1

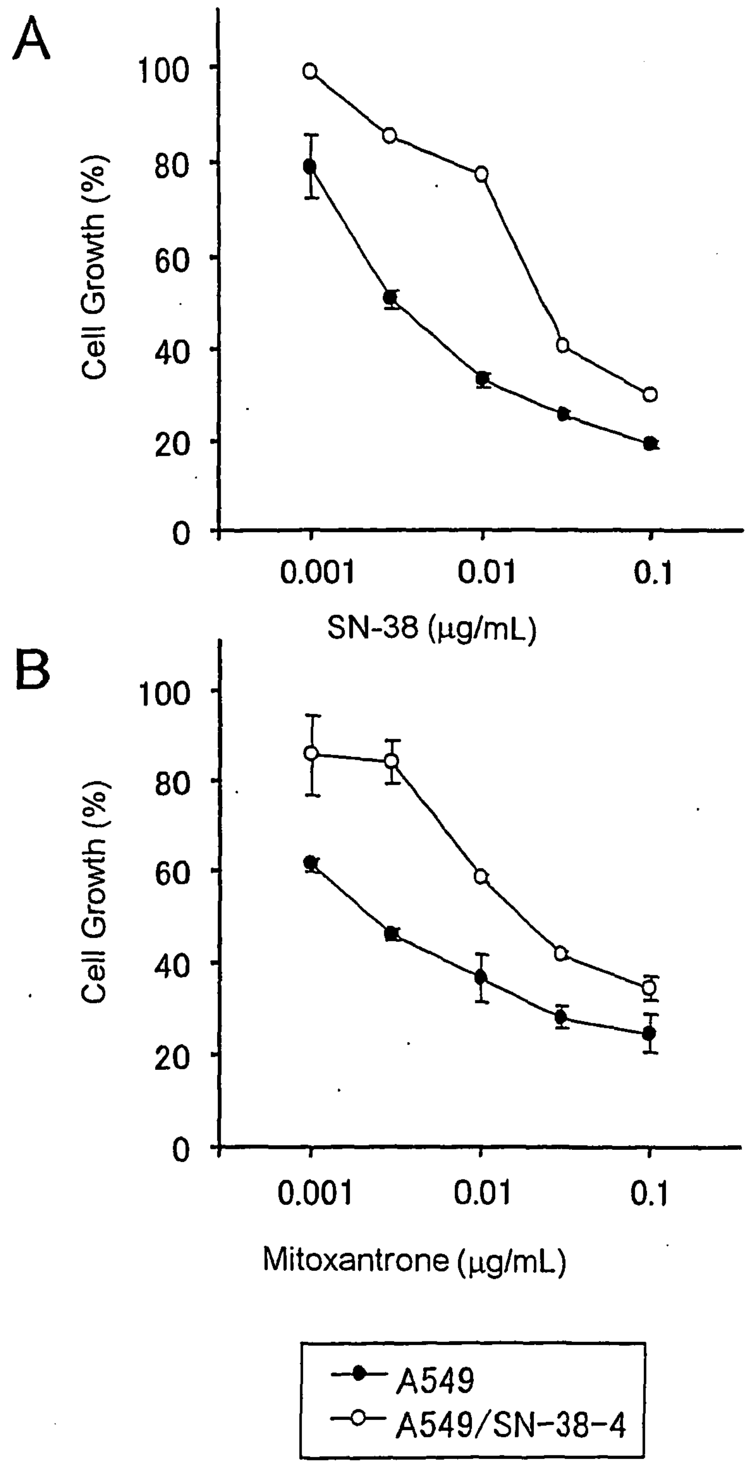
A
100
80
60
40
20
0
Cell Growth (%)
0.001
0.01
0.1
SN-38 (μg/mL)
B
100
80
60
40
20
0
Cell Growth (%)
0.001
0.01
0.1
Mitoxantrone (μg/mL)
A549
A549/SN-38-4

Fig. 2

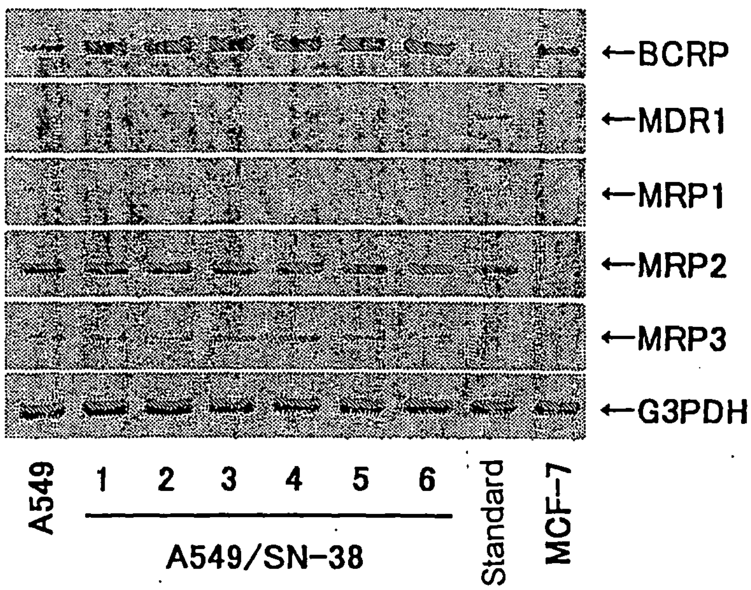
←BCRP
←MDR1
←MRP1
←MRP2
←MRP3
←G3PDH
A549
1
2
3
4
5
6
A549/SN-38
Standard
MCF-7

Fig. 3

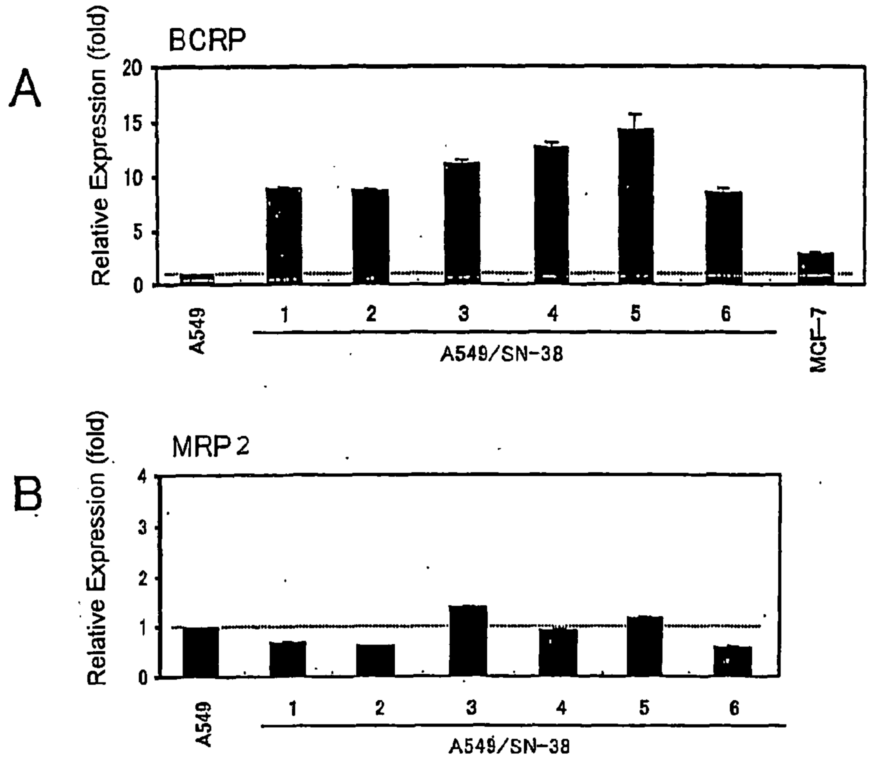
A
BCRP
Relative Expression (fold)
20
15
10
5
0
A549
1
2
3
4
5
6
A549/SN-38
MCF-7
B
MRP 2
Relative Expression (fold)
4
3
2
1
0
A549
1
2
3
4
5
6
A549/SN-38

Fig. 4

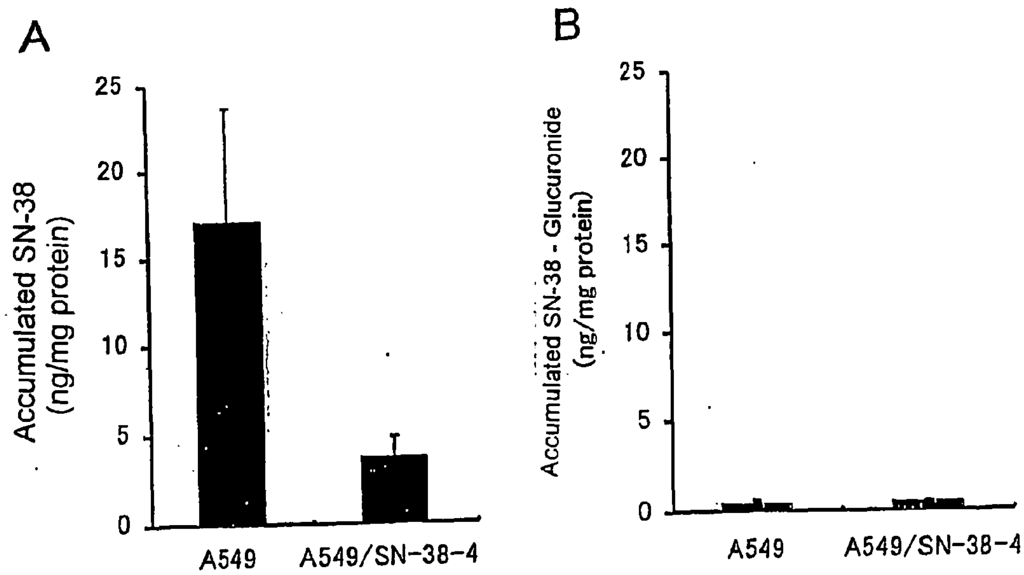
A
Accumulated SN-38
(ng/mg protein)
25
20
15
10
5
0
A549
A549/SN-38-4
B
Accumulated SN-38 - Glucuronide
(ng/mg protein)
25
20
15
10
5
0
A549
A549/SN-38-4

Fig. 5

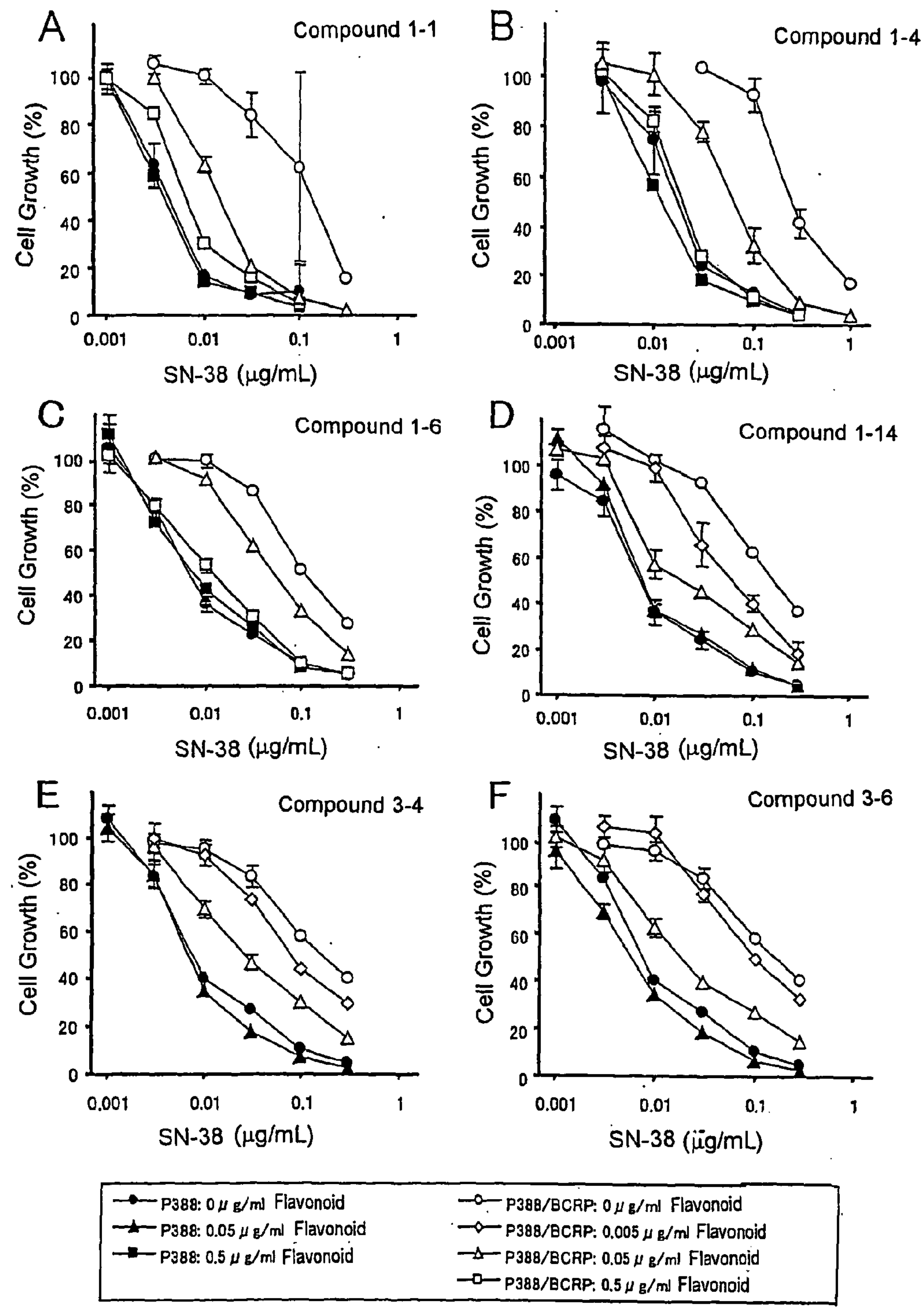
A
Compound 1-1
B
Compound 1-4
C
Compound 1-6
D
Compound 1-14
E
Compound 3-4
F
Compound 3-6
Cell Growth (%)
SN-38 (μg/mL)
0
20
40
60
80
100
0.001
0.01
0.1
1
P388: 0 μg/ml Flavonoid
P388: 0.05 μg/ml Flavonoid
P388: 0.5 μg/ml Flavonoid
P388/BCRP: 0 μg/ml Flavonoid
P388/BCRP: 0.005 μg/ml Flavonoid
P388/BCRP: 0.05 μg/ml Flavonoid
P388/BCRP: 0.5 μg/ml Flavonoid

Fig. 6

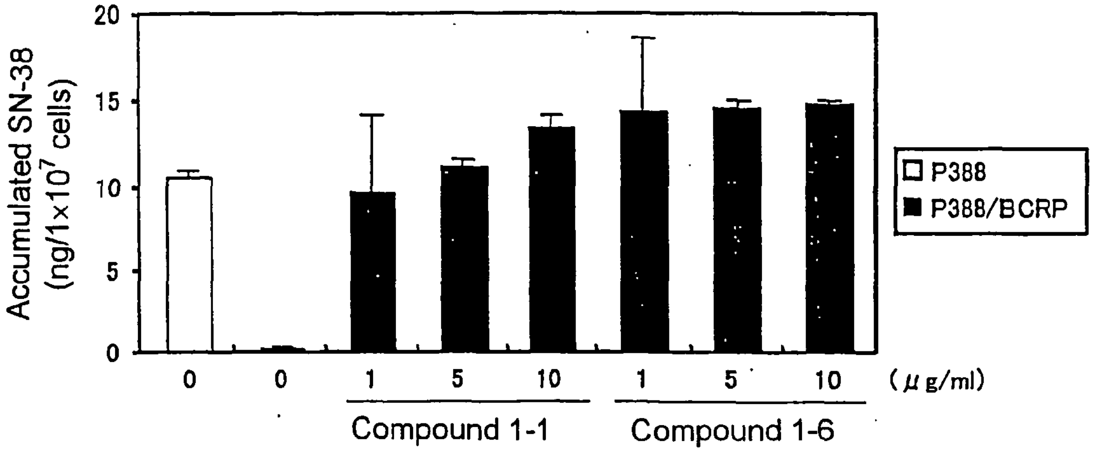
Accumulated SN-38 (ng/1×10^7 cells)
20
15
10
5
0
0
0
1
5
10
1
5
10
(μg/ml)
Compound 1-1
Compound 1-6
P388
P388/BCRP

Fig. 7

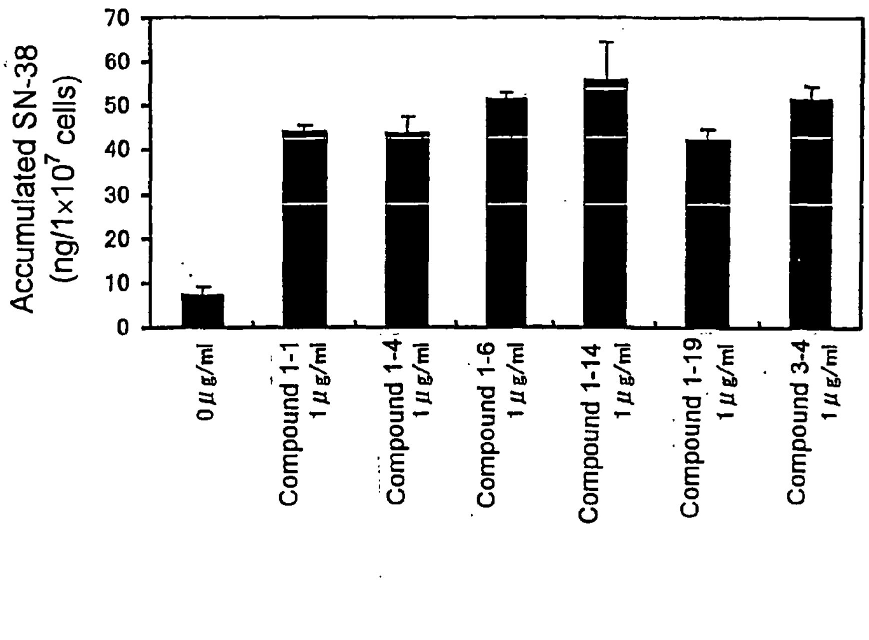
Accumulated SN-38 (ng/1×10^7 cells)
70
60
50
40
30
20
10
0
0μg/ml
Compound 1-1 1μg/ml
Compound 1-4 1μg/ml
Compound 1-6 1μg/ml
Compound 1-14 1μg/ml
Compound 1-19 1μg/ml
Compound 3-4 1μg/ml

**INTERNATIONAL SEARCH REPORT**

International application No.
PCT/JP2004/001054

**A. CLASSIFICATION OF SUBJECT MATTER**

Int.Cl[7] A61K31/121, 31/352, A61P35/00, 43/00, C07D311/30, 493/04, C07H17/07, C12N5/08, C12Q1/02

According to International Patent Classification (IPC) or to both national classification and IPC

**B. FIELDS SEARCHED**

Minimum documentation searched (classification system followed by classification symbols)
Int.Cl[7] A61K31/121, 31/352, A61P35/00, 43/00, C07D311/30, 493/04, C07H17/07, C12N5/08, C12Q1/02

Documentation searched other than minimum documentation to the extent that such documents are included in the fields searched

Electronic data base consulted during the international search (name of data base and, where practicable, search terms used)
CAPLUS(STN), REGISTRY(STN), MEDLINE(STN), BIOSIS(STN), EMBASE(STN)

**C. DOCUMENTS CONSIDERED TO BE RELEVANT**

| Category* | Citation of document, with indication, where appropriate, of the relevant passages | Relevant to claim No. |
|---|---|---|
| X<br>A | JP 54-70348 A (Kureha Chemical Industry Co., Ltd.),<br>23 March, 1993 (23.03.93),<br>Full text<br>(Family: none) | 1-3<br>4-6 |
| X | WO 00/19998 A1 (KGK SYNERGINE),<br>13 April, 2000 (13.04.00),<br>Full text<br>& EP 1119353 A1 & JP 2003-509334 A | 2,3 |
| X | JP 2001-342132 A (Protein Technologies International, Inc.),<br>11 December, 2001 (11.12.01),<br>Full text<br>& AU 2000-48794 A | 2,3 |

[X] Further documents are listed in the continuation of Box C. [ ] See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 23 March, 2004 (23.03.04) | 06 April, 2004 (06.04.04) |
| Name and mailing address of the ISA/<br>Japanese Patent Office | Authorized officer |
| Facsimile No. | Telephone No. |

Form PCT/ISA/210 (second sheet) (January 2004)

**INTERNATIONAL SEARCH REPORT**

International application No. PCT/JP2004/001054

C (Continuation). DOCUMENTS CONSIDERED TO BE RELEVANT

| Category* | Citation of document, with indication, where appropriate, of the relevant passages | Relevant to claim No. |
|---|---|---|
| X | EP 1159963 A1 (Protein Technologies International, Inc.), 05 December, 2001 (05.12.01), Full text & JP 2001-335484 A & US 6300367 B1 | 2,3 |
| P,X | JP 2003-40781 A (Ezaki Glico Co., Ltd.), 13 February, 2003 (13.02.03), Full text (Family: none) | 2,3 |
| X | EP 74256 A1 (KABUSHIKI KAISHA YAKULUT HONSHA), 16 March, 1983 (16.03.83), Particularly, page 29, example 7(B) & JP 58-39683 A | 7 |
| A | WO 99/43676 A2 (HOECHST MARION ROUSSEL, INC.), 02 September, 1999 (02.09.99), Particularly, page 18, line 11 & JP 2002-504553 A | 7 |
| A | DOYLE L.A. et al., A multidrug resistance transporter from human MCF-7 breast cancer cells, Proc.Natl.Acad.Sci.USA, 1998, Vol.95, No.26, pages 15665 to 15670 | 1-7 |
| A | BRANGI, M. et al., Camptothecin Resistance:Role of the ATP-binding Cassette (ABC), Mitoxantrone-resistance Half-Transporter (MXR), and Potential for Glucuronidation in MXR-expressing Cells, Cancer Res., 1999, Vol.59, No.23, pages 5938 to 5946 | 1-7 |
| A | WO 99/40110 A1 (UNIVERSITY OF MARYLAND, BALTIMORE), 12 August, 1999 (12.08.99), Full text & EP 1054894 A1 & US 6313277 B1 & JP 2002-502592 A | 1-7 |

Form PCT/ISA/210 (continuation of second sheet) (January 2004)

**INTERNATIONAL SEARCH REPORT**

International application No. PCT/JP2004/001054

**Box No. II Observations where certain claims were found unsearchable (Continuation of item 2 of first sheet)**

This international search report has not been established in respect of certain claims under Article 17(2)(a) for the following reasons:

1. ☐ Claims Nos.: because they relate to subject matter not required to be searched by this Authority, namely:

2. ☐ Claims Nos.: because they relate to parts of the international application that do not comply with the prescribed requirements to such an extent that no meaningful international search can be carried out, specifically:

3. ☐ Claims Nos.: because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

The inventions as set forth in claims are composed of 2 groups of inventions, i.e., (1 to 6) and (7).

Although claims 1 to 7 each relates to BCRP, BCRP is a publicly known protein as approved by the applicant. Thus, it cannot be considered as "a special technical feature" in the meaning within PCT Rule 13.2.

The special technical features in claims 1 to 6 reside in flavonoid compounds or breast cancer-resistant protein inhibitors, anticancer drug-resistance overcoming agents or anticancer drug potentiators containing these compounds.

On the other hand, the special technical feature of claim 7 resides in an SN-38-resistant human non-small cell lung cancer (continued to extra sheet)

1. ☐ As all required additional search fees were timely paid by the applicant, this international search report covers all searchable claims.

2. ☒ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest.

☐ No protest accompanied the payment of additional search fees.

Form PCT/ISA/210 (continuation of first sheet (2)) (January 2004)

**INTERNATIONAL SEARCH REPORT**

International application No.
PCT/JP2004/001054

Continuation of Box No.III of continuation of first sheet(2)

A549 cell with overexpression of BCRP.

Thus, it does not appear that there is a technical relationship between these groups of inventions involving one or more of the same or corresponding special technical features. Such being the case, these groups of inventions are not considered as relating to a group of inventions so linked as to form a single general inventive concept.

Form PCT/ISA/210 (extra sheet) (January 2004)